# EUROPÄISCHE PATENTSCHRIFT

(11) **EP 2 152 687 B1**
(45) Veröffentlichungstag und Bekanntmachung des Hinweises auf die Patenterteilung: **19.01.2011**
(21) Anmeldenummer: 08759840.5
(22) Anmeldetag: 21.05.2008
(51) Int. Cl.: C07D 317/34

(54) **VERFAHREN ZUR HERSTELLUNG CARBONAT-TERMINIERTER VERBINDUNGEN**
METHOD FOR PRODUCING CARBONATE-TERMINATED COMPOUNDS
PROCÉDÉ DE FABRICATION DE COMPOSÉS À TERMINAISON CARBONATE

(30) Priorität: 21.05.2007 DE 102007023867
(43) Veröffentlichungstag der Anmeldung: 17.02.2010
(73) Patentinhaber: Saltigo GmbH, 40764 Langenfeld (DE)
(72) Erfinder: JOB, Andreas, 50858 Köln (DE); MÖLLER, Martin, 52070 Aachen (DE); HE, Yingchun, 52074 Aachen (DE); PASQUIER, Nicolas, Shanghai 200135 (CN); MENDREK, Aleksandra, 52074 Aachen (DE); KEUL, Helmut, 52074 Aachen (DE)
(74) Vertreter: Wichmann, Birgid
(86) Internationale Anmeldenummer: PCT/EP2008/056236
(87) Internationale Veröffentlichungsnummer: WO 2008/142097

(56) Entgegenhaltungen:
- EP-A- 0 289 842
- EP-A- 1 541 568

## Beschreibung

Die vorliegend Erfindung betrifft ein Verfahren zur Herstellung Carbonat-terminierter Verbindungen.

Für eine Vielzahl von Materialien und Anwendungen ist die dauerhafte Funkmionalisierung von Oberflächen von Bedeutung. Materialoberflächen können zum Beispiel durch Beschichtung mit Heparin biokompatibel gemacht werden. Andere Anwendungen sind schmutzabweisende und bakteriostatische Ausrüstungen und die Verbesserung der Haftung von Klebstoffen und Lacken.

Auch die gezielte Modifizierung von Biomolekülen ist in vielen Fällen wünschenswert. So ist es beispielsweise bekannt, dass die biologische Halbwertszeit verschiedener Wirkstoffe durch Anfügen von Polyoxyalkylenresten verbessert werden kann.

Aus EP-A-1 541 568 sind beispielsweise reaktive cyclische Carbonate der allgemeinen Formel 1 bekannt, 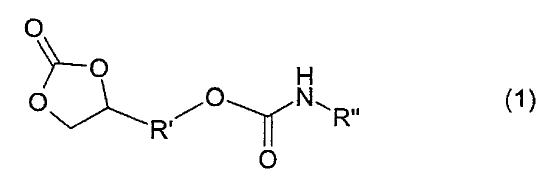 wobei R' tür C₁-C₁₂ Alkylen steht und R" verschiedenste organische Reste darstellen kann. Diese reaktiven cyclischen Carbonate lassen sich mit Nucleophilen, z.B. Hydroxy- oder primären oder sekundären Aminogruppen von Biomolekülen, Polymeren oder an Substratoberflächen umsetzen. Hierbei kommt es zur Öffnung des cyclischen Carbonatrings unter gleichzeitiger kovalenter Anbindung an das Biomolekül, das Polymer oder sonstige Substratoberflächen.

Die Herstellung der reaktiven cyclischen Carbonate der Formel (1) erfolgt dabei durch Umsetzung der phenylsubstituierten cyclischen Carbonate der allgemeinen Formel (2), 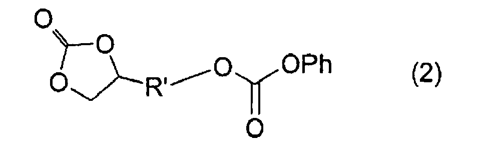 mit Aminen der allgemeinen Formel (3)

H₂N-R" (3)

Nachteilig ist bei dieser Synthese die Freisetzung von Phenol als Nebenprodukt der Substitution mit dem Amin in äquimolaren Mengen. Je nach erhaltenem Reaktionsprodukt der Formel (1) ist die Abtrennung des toxikologisch problematischen Phenols mit hohem Aufwand verbunden.

Die US-A-5,115,045 betrifft Isocyanat-reaktive Zusammensetzungen, die zur Herstellung von Polyurethanen eingesetzt werden. Diese Isocyanat-reaktiven Zusammensetzungen basieren auf dem Umsetzungsprodukt von (a) einem Di- oder Polychlorformiatester des Typs Q-[X-COY]ₙ, wobei X Sauerstoff und Y Chlor bedeuten kann, n mindestens 2 ist und Q einen organischen Rest darstellt, mit (b) einer mehrfach-funktionellen Verbindung, die neben den funktionellen Gruppen, welche mit dem Di- oder Polychlorformiatester (a) abreagieren, zwingend mindestens eine Imino- oder Enaminogruppe enthalten muss. Es werden keine Di- oder Polychlorformiatester (a) beschrieben, die cyclische Carbonatgruppen aufweisen. Die Herstellung der Di- oder Polychlorformiatester erfolgt durch Umsetzung von Polyolen mit Acylierungsmitteln wie Phosgen. Aus den Iminound/oder Enaminogruppen, die in der mehrfach-funktionellen Verbindung (b) enthalten sind, kann später durch Hydrolyse eine gegenüber Isocyanaten reaktive Aminogruppe erhalten werden.

Da ein hoher Bedarf nach reaktiven cyclischen Carbonaten zur Oberflächenbehandlung besteht, lag dieser Erfindung die Aufgabe zugrunde, ein verbessertes Herstellverfahren bereitzustellen.

**Gegenstand der Erfindung** ist ein Verfahren zur Herstellung von Verbindungen der allgemeinen Formel (I), 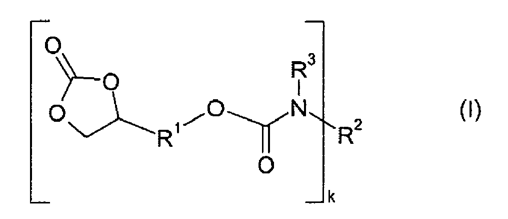 worin
- R¹: C₁-C₁₂-Alkylen bedeutet,
- k: für 1 oder eine ganze Zahl größer als 1 steht,
und wobei, wenn k für 1 steht,
- R² und R³: gleich oder verschieden sind und für C₁-C₃₀-Alkyl, C₂-C₃₀-Alkenyl, C₁-C₃₀- Halogenalkyl, C₁-C₃₀-Hydroxyalkyl, C₁-C₆-Alkyloxy-C₁-C₃₀-alkyl, C₁-C₆- Alkylcarbonyloxy-C₁-C₃₀-alkyl, Amino-C₁-C₃₀-alkyl, Mono- oder Di(C₁-C₁₈- alkyl)amino-C₁-C₃₀-alkyl, Mono- oder Di(C₇-C₁₁-aralkyl)amino-C₁-C₃₀-alkyl, Mono- oder Di(allyl)amino-C₁-C₃₀-alkyl, Ammonio-C₁-C₃₀-alkyl, Polyoxyalkylen- C₁-C₃₀-alkyl, Polysiloxanyl-C₁-C₃₀-alkyl, (Meth)acryloyloxy-C₁-C₃₀-alkyl, Sulfono-C₁-C₃₀-alkyl, Phosphono-C₁-C₃₀-alkyl, Di(C₁-C₆-alkyl)phosphono-C₁-C₃₀- alkyl, Phosphonato-C₁-C₃₀-alkyl, Di(C₁-C₆-alkyl)phosphonato-C₁-C₃₀-alkyl, 3- Imidazolio-C₁-C₃₀-Alkyl, -[C₂-C₆-Alkylen-O]ₓ-[C₂-C₆-Alkylen-O]_{y}-R, wobei R für Wasserstoff oder C₁-C₆-Alkyl steht, x = 0-50 und y = 0-50 ist, entweder x oder y R³ mindestens 1 ist und die beiden Alkylenreste verschieden sind, wenn x und y beide ungleich 0 sind, oder einen Saccharidrest steht, und statt der vorgenannten Bedeutungen alternativ auch für Wasserstoff stehen kann, oder aber
- R² und R³: unter Einbeziehung des Stickstoffatoms, an das sie gebunden sind, einen cyclischen aliphatischen oder aromatischen Rest bilden, der 4 oder 5 Kohlenstoffatome aufweist, wobei ein oder zwei dieser Kohlenstoffatome zusätzlich durch Heteroatome, ausgewählt aus Stickstoff, Schwefel und Sauerstoff, ersetzt sein können, oder aber
- R³: für Wasserstoff steht und gleichzeitig
- R²: für einen Fluoresceinyl-Rest oder einen über eine C₁-C₃₀-Alkylengruppe gebundenen Pyren-, Fluorescein-, Cumarin-, 4,4'-Bisstyrylbiphenyl-, Pyrazolin-, Hydrochinolon-, Benzoxazol-, Benzisoxazol-, Benzimidazol- oder Flavonsäure- Rest steht,
und wobei, wenn k für eine ganze Zahl größer als 1 steht,
- R³: die gleichen Bedeutungen wie für k=1 besitzt und
- R²: für einen k-valenten aliphatischen, cycloaliphatischen, aromatischen oder araliphatischen Kohlenwasserstoff-Rest steht, der gegebenenfalls ein oder mehrere Heteroatome enthält,
durch Umsetzung von Verbindungen der allgemeinen Formel (II) 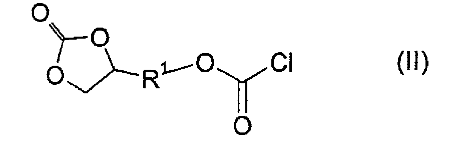 worin R¹ die für die allgemeine Formel (I) genannte Bedeutung hat,
mit einer Verbindung der allgemeinen Formel (III), 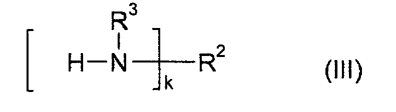 worin
k und ebenso R² und R³ in Abhängigkeit von k die für die allgemeine Formel (I) beschriebenen Bedeutungen haben,
oder einem Salz davon.

Das erfindungsgemäße Verfahren zeichnet sich dadurch aus, dass bei der Umsetzung kein Phenol als Nebenkomponente entsteht. Ferner werden sehr gute Ausbeuten erhalten, und vorteilhafterweise bleibt der cyclische Carbonat-Ring im Verlauf der erfindungsgemäßen Reaktion erhalten und wird nicht in einer Nebenreaktion geöffnet.

Sofern in dieser Anmeldung der Begriff "substituiert" verwendet wird, so bedeutet dies im Sinne dieser Anmeldung, dass ein Wasserstoff-Atom an einem Rest oder Atom durch eine der angegebenen Gruppen ersetzt ist, mit der Maßgabe, dass die Wertigkeit des angegebenen Atoms nicht überschritten wird und die Substitution zu einer stabilen Verbindung führt.

Im Rahmen dieser Anmeldung und Erfindung können alle zuvor genannten und im Folgenden genannten, allgemeinen oder in Vorzugsbereichen genannten Definitionen von Resten, Parametern oder Erläuterungen untereinander, also auch zwischen den jeweiligen Bereichen und Vorzugsbereichen in beliebiger Weise kombiniert werden.

Der Begriff "Alkyl" soll geradkettige, verzweigte und cyclische Alkylgruppen umfassen. Sofern in dieser Anmeldung nicht explizit anders angegeben, besitzen diese Alkylgruppen jeweils 1 bis 30, bevorzugt 1 bis 24 und besonders bevorzugt 1 bis 18 Kohlenstoffatome. Geeignet sind als Alkylreste beispielsweise Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl, n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl.

Der Begriff **"Halogenalkyl"** bedeutet entsprechend eine Alkylgruppe der vorgenannten Bedeutung, in der eine oder mehrere Wasserstoffatome durch Halogen, vorzugsweise Chlor oder Fluor, ersetzt sind.

Der Begriff **"Hydroxyalkyl"** bedeutet entsprechend eine Alkylgruppe der oben genannten Bedeutung, in der ein oder mehrere Wasserstoffatome durch Hydroxygruppen ersetzt sind.

In gleicher Weise bedeuten im Rahmen der Definitionen der Reste R² und R³ die Begriffe "Alkyloxyalkyl", "Alkylcarbonyloxyalkyl", "Aminoalkyl", "Mono- oder Di(alkyl)aminoalkyl", "Mono- oder Di(aralkyl)amino-alkyl", "Mono- oder Di(allyl)amino-alkyl", "Ammonioalkyl", "Polyoxyalkylenalkyl", "Polysiloxanylalkyl"_{,} "(Meth)acryloyloxyalkyl", "Sulfonoalkyl", "Phosphonoalkyl" und "Dialkylphophonoalkyl", "Phosphonatoalkyl", "Dialkylphosphonatoalkyl" und "Imidazolioalkyl" einen Alkylrest, in dem ein oder mehrere Wasserstoffatome durch eine Alkyloxy-Gruppe, eine Alkylcarbonyloxy-Gruppe, eine Amino-Gruppe, eine Mono- oder Dialkylamino-Gruppe, eine Ammonio-Gruppe ((-NR⁵₃)⁺, worin R⁵ unabhängig voneinander z.B. für C₁-C₁₈-Alkyl, C₂-C₁₈-Alkenyl oder Benzyl stehen können), einen Polyoxyalkylen-Rest, einen Polysiloxanyl-Rest, eine (Meth)acryloyloxy-Gruppe, eine Sulfonsäure-Gruppe (-SO₃H), eine Phosphonsäure-Gruppe (-PO₃H₂), Phosphorsäureester-Gruppe (-OPO₃H₂) bzw. eine Imidazolio-Gruppe der folgenden Struktur 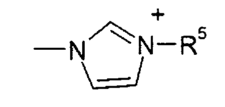 worin R⁵ Wasserstoff, C₁-C₁₈ Alkyl, C₂-C₁₈ Alkenyl, insbesondere Allyl, oder Benzyl bedeutet, ersetzt sind. Wenn R² und/oder R³ für Ammonioalkyl oder Imidazolioalkyl stehen, ist die Verbindung von einem Äquivalent eines Anions, vorzugsweise eines physiologisch verträglichen Anions, wie eines Halogenids, insbesondere Chlorid oder Bromid, Sulfat, Hydrogensulfat, Methosulfat oder Nitrat begleitet.

Der Begriff **"Alkenyl"** bedeutet eine Alkylgruppe mit 2 bis 30, vorzugsweise 2 bis 24 und insbesondere 2 bis 18 Kohlenstoffatomen, enthält jedoch eine oder mehrere mittel- oder auch endständige C=C Doppelbindungen.

Der Begriff **"Alkylen"** bedeutet einen divalenten aliphatischen, verzweigten oder unverzweigten Kohlenwasserstoffrest, vorzugsweise einen -(CH₂-CH₂)-, einen -(CH₂-CH(CH₃))-, einen -(CH₂-CH(C₂H₅))- oder einen -(CH₂-CH₂-CH₂-CH₂)- Rest.

Der **Polyoxyalkylenrest** leitet sich vorzugsweise von Ethylenoxid, Propylenoxid, Butylenoxid oder Tetrahydrofuran ab, besonders bevorzugt von Ethylenoxid oder Propylenoxid. Er ist am distalen Ende durch einen Rest terminiert, bei dem es sich um Wasserstoff oder einen C₁-C₆-Alkyl-Rest handelt.

Der **Polysiloxanylrest** leitet sich vorzugsweise von Polydimethylsiloxanen ab und kann geradkettig oder verzweigt sein.

Bei dem Saccharidrest handelt es sich z.B. um einen Glucoxylrest.

Im erfindungsgemäßen Verfahren werden bevorzugt Verbindungen der allgemeinen Formel (II) eingesetzt, worin R¹ einen C₁-C₆-Alkylen-Rest, besonders bevorzugt einen Methylen-, Ethylen-, n-Propylen, n-Butylen- oder n-Hexamethylenrest darstellt.

Sofern im erfindungsgemäßen Verfahren Verbindungen der allgemeinen Formel (III) eingesetzt werden, worin k gleich 1 ist, R³ Wasserstoff bedeutet und R² für einen Fluoresceinyl-Rest oder einen über eine C₁-C₃₀-Alkylengruppe gebundenen Pyren-, Fluorescein-, Cumarin-, 4,4'-Bisstyrylbiphenyl-, Pyrazolin-, Hydrochinolon-, Benzoxazol-, Benzisoxazol-, Benzimidazol- oder Flavonsäure-Rest steht, so können die vorgenannten Reste R² auch ein- oder mehrfach substituiert sein. Beispielhaft seien hier die Substitutionsmuster genannt, die in der WO-A-2005/108961 beschrieben sind.

Im erfindungsgemäßen Verfahren können die Verbindungen der allgemeinen Formel (III) alternativ auch in Form ihrer jeweiligen Ammoniumsalze eingesetzt werden, welche die allgemeine Formel (IIIA) besitzen, 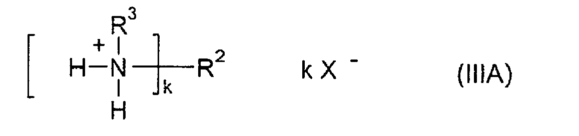 wobei R² und R³ jeweils die in dieser Anmeldung im Zusammenhang mit der Formel (III) genannten Bedeutungen besitzen und X für ein Halogenid, bevorzugt Chlor, Brom oder Iod, oder Methylsulfat steht.

Geeigneterweise werden Verbindungen der allgemeinen Formel (III) eingesetzt, bei denen
- k: gleich 1 ist und
- R² und R³: gleich oder verschieden sind und für C₁-C₃₀-Alkyl, C₁-C₆-Alkyloxy-C₁-C₃₀-alkyl, C₁-C₆-Alkylcarbonyloxy-C₁-C₃₀-alkyl, Amino-C₁-C₃₀-alkyl, Mono- oder Di(C₁-C₆- alkyl)amino-C₁-C₃₀-alkyl, Mono- oder Di(C₇-C₁₁-aralkyl)amino-C₁-C₃₀-alkyl, Mono- oder Di(allyl)amino-C₁-C₃₀-alkyl, Ammonio-C₁-C₃₀-alkyl, Polyoxyalkylen- C₁-C₃₀-alkyl, Polysiloxanyl-C₁-C₃₀-alkyl, (Meth)acryloyloxy-C₁-C₃₀-alkyl, 3- Imidazolio-C₁-C₃₀-Alkyl, oder [C₂-C₆-Alkylen-O]ₓ-[C₂-C₆-Alkylen-O]_{y}-C₁-C₆- Alkyl, wobei x = 0-50 und y = 0-40 ist, entweder x oder y mindestens 1 ist und die beiden Alkylenreste verschieden sind, sofern x und y beide ungleich 0 sind, oder
- R³: alternativ auch für Wasserstoff stehen kann.

Geeignet sind auch Verbindungen der allgemeinen Formel (III), bei denen
- k: gleich 1 ist
- R²: für C₁-C₁₈-Alkyl, C₁-C₆-Alkyloxy-C₁-C₁₈-alkyl, C₁-C₆-Alkylcarbonyloxy-C₁-C₁₈- alkyl, Amino-C₁-C₁₈-alkyl, Mono- oder Di(C₁-C₆-alkyl)amino-C₁-C₁₈-alkyl, Mono- oder Di(C₇-C₁₁-aralkyl)amino-C₁-C₁₈-alkyl, Mono- oder Di(allyl)amino-C₁-C₁₈- alkyl, Ammonio-C₁-C₁₈-alkyl, Polyoxyalkylen-C₁-C₁₈-alkyl, Polysiloxanyl-C₁-C₁₈- alkyl, (Meth)acryloyloxy-C₁-C₁₈-alkyl, 3-Imidazolio-C₁C₃₀-Alkyl, oder [C-₂-C₆- Alkylen-O]ₓ-[C₂-C₆-Alkylen-O]_{y}-C₁-C₆-Alkyl, wobei die beiden Alkylenreste verschieden sind und x = 1-50 und y = 1-40, steht und
- R³: Wasserstoff bedeutet.

Ebenfalls geeignet sind Verbindungen der allgemeinen Formel (III), bei denen
- k: gleich 1 ist und
- R² und R³: gleich oder verschieden sind und für C₁-C₁₈-Alkyl, Amino-C₁-C₁₈-alkyl, Mono- oder Di(C₁-C₆-alkyl)amino-C₁-C₁₈-alkyl, Mono- oder Di(C₇-C₁₁-aralkyl)amino-C₁- C₁₈-alkyl, Mono- oder Di(allyl)amino-C₁-C₁₈-alkyl, Ammonio-C₁-C₁₈-alkyl, Polyoxyalkylen-C₁-C₁₈-alkyl, Polysiloxanyl-C₁-C₁₈-alkyl, 3-Imidazolio-C₁-C₃₀- Alkyl oder [C₂-C₆-Alkylen-O]ₓ-[C₂-C₆-Alkylen-O]_{y}-C₁-C₆-Alkyl, wobei die beiden Alkylenreste verschieden sind und x = 2-40 und y = 2-20 ist, stehen.

Weiterhin können Verbindungen der allgemeinen Formel (III) eingesetzt werden, bei denen
- k: gleich 1 ist,
- R³: Wasserstoff bedeutet und R²
für -(CH₂)ₙ-CH₃,
-(CH₂)ₙCH((CH₂)ₘ-CH₃)-(CH₂)ₒ-CH₃, wobei n+m+o maximal 27 ergeben,
-(CH₂)ₙ-(CF₂)ₚ-CF₃,
-(CH₂)ₙ-[O-Si(-O-Si(CH₃)₃)₃],
-(CH₂)ₙ-(O-CH₂-CH₂)_{q}-O-R⁴-H,
-R⁴-OH,
-R⁴-NR⁵₂,
-R⁴-NR⁵₃⁺ Y⁻,
-R⁴-OC(=O)-CR⁶=CH₂,
-R⁴-SO₃H,
-R⁴-PO₃H₂,
-R⁴-OPO₃H₂
oder einen Saccharidrest steht,
worin
R⁴ für C₁-C₆-Alkylen,
R⁵ für Wasserstoff, C₁-C₁₈ Alkyl, C₂-C₁₈ Alkenyl, bevorzugt Allyl, oder Benzyl, R⁶ für Wasserstoff oder Methyl und
Y für ein Äquivalent eines Anions, bevorzugt Chlorid, Bromid, Sulfat, Hydrogensulfat, Methosulfat oder Nitrat steht, n, m,o unabhängig voneinander eine ganze Zahl von 0 bis 17 sind,
p eine ganze Zahl von 1 bis 12 ist und
q für eine ganze Zahl von 1 bis 100, bevorzugt 2 bis 50 steht.

Im erfindungsgemäßen Verfahren können ferner Verbindungen der allgemeinen Formel (III) eingesetzt werden, bei denen
- k: eine ganze Zahl größer 1, insbesondere 2 oder 3 ist,
- R²: für einen k-valenten, insbesondere di- oder trivalenten, aliphatischen, cycloaliphatischen, aromatischen oder araliphatischen Kohlenwasserstoff-Rest steht, der gegebenenfalls ein oder mehrere Heteroatome enthält, und
- R³: für Wasserstoff, C₁-C₃₀-Alkyl, C₁-C₆-Alkyloxy-C₁-C₃₀-alkyl, C₁-C₆- Alkylcarbonyloxy-C₁-C₃₀-alkyl, Amino-C₁-C₃₀-alkyl, Mono- oder Di(C₁-C₆-alkyl)- amino-C₁-C₃₀-alkyl, Mono- oder Di(C₇-C₁₁-aralkyl)amino-C₁-C₃₀-alkyl, Mono- oder Di(allyl)amino-C₁-C₃₀-alkyl, Ammonio-C₁-C₃₀-alkyl, Polyoxyalkylen-C₁-C₃₀- alkyl, Polysiloxanyl-C₁-C₃₀-alkyl, (Meth)acryloyloxy-C₁-C₃₀-alkyl, 3-Imidazolio- C₁-C₃₀-Alkyl oder [C₂-C₆-Alkylen-O]ₓ-[C₂-C₆-Alkylen-O]_{y}-C₂-C₆-Alkyl, wobei x = 0-50 und y = 0-50 ist, aber entweder x oder y mindestens 1 ist und die beiden Alkylenreste verschieden sind, wenn x und x beide ungleich 0 sind, steht.

Bevorzugt werden Verbindungen der allgemeinen Formel (III) eingesetzt, bei denen
- k: 2 oder 3 ist,
- R²: für einen di- oder trivalenten, aliphatischen, cycloaliphatischen, aromatischen oder araliphatischen Kohlenwasserstoff-Rest steht, der gegebenenfalls ausschließlich ein oder mehrere Sauerstoffatome als Heteroatome enthält, und
- R³: für Wasserstoff, C₁-C₁₈-Alkyl, C₁-C₆-Alkyloxy-C₁-C₁₈-alkyl, C₁-C₆- Alkylcarbonyloxy-C₁-C₁₈-alkyl, Amino-C₁-C₁₈-alkyl, Mono- oder Di(C₁-C₆-alkyl)- amino-C₁-C₁₈-alkyl, Ammonio-C₁-C₁₉-alkyl, Polyoxyalkylen-C₁-C₁₈-alkyl, Polysiloxanyl-C₁-C₁₈-alkyl, (Meth)acryloyloxy-C₁-C₁₈-alkyl, 3-Imidazolio-C₁-C₃₀- Alkyl oder [C₂-C₆-Alkylen-O]ₓ-[C₂-C₆-Alkylen-O]_{y}-C₂-C₆-Alkyl, wobei x = 1-50 und y = 1-50 ist und die beiden Alkylenreste verschieden sind, steht.

Besonders bevorzugt werden ferner Verbindungen der allgemeinen Formel (III) eingesetzt, bei denen
- k: 2 ist,
- R²: für eine -[(C₂-C₆-Alkylen-O-)ₓ-(C₂-C₆-Alkylen)]- Gruppe steht, worin die beiden Alkylengruppen gleich oder verschieden sind und x = 1-50, bevorzugt x=1-40 ist, oder für eine -[(C₂-C₆-Alkylen-O-)ₓ-(C₂-C₆-Alkylen-O-)_{y}-(C₂-C₆-Alkylen-O-)_{z}-C₂-C₆-Alkylen]- Gruppe steht, wobei die drei Alkyleneinheiten verschieden sind bzw. die erste mit der dritten identisch sein kann, und wobei x = 0-50, inbesondere x = 1-40, y = 1- 50, insbesondere y = 1-40, und z = 1-50, insbesondere z = 1-40 ist, und
- R³: Wasserstoff oder C₁-C₃₀-Alkyl bedeutet.

Die Amine der allgemeinen Formel (III) sind entweder käuflich erhältlich oder aber nach dem Fachmann geläufigen Methoden herstellbar.

Wenn k = 1 ist, können im erfindungsgemäßen Verfahren beispielsweise die folgenden Amine der allgemeinen Formel (III) eingesetzt werden:
Polyetheramine, n-Octylamin, tert-Octylamin, n-Hexylamin, 2-Ethylhexylamin, Taurin, Dihexylamin, Dodecylamin, Octadecylamin, Methyloctadecylamin, Dioctylamin, N,N-Dimethylpropylendiamin, N,N-Dimethylethylendiamin, 3-Aminopropyltrimethylammoniumiodid, 3-Aminopropyltrimethylammoniumbromid, 3-Aminopropyltrimethylammoniumchlorid, 3-Aminoethyltrimethylammoniumiodid, 3-Aminoethyltrimethylammoniumbromid, 3-Aminoethyltrimethylammoniumchlorid, 3-Aminopropyldimethyloctylammoniumchlorid, 3-Aminopropyldimethyloctylammoniumbromid, 3-(Trimethylsilyl)-propylamin, 3-(Tris(trimethyisilyloxy)silyl)-propylamin, 3-(Trimethylsilyl)-ethylamin, 3-(Tris(trimethylsilyloxy)silyl)-ethylamin, 3-Aminopropylimidazol, 2-Aminoethylimidazol, 4-Aminobutylimidazol, 3-Aminopropyl-1-methylimidiazolium-methylsulfat, 3-Aminopropyl-1-butylimidiazoliummethylsulfat, 3-Amino-ethyl-1-methyl-imidiazolium-methylsulfat, 3-Aminoethyl-1-butylimidiazoliummethylsulfat, 3-Aminopropyl-1-methylimidazoliumchlorid, 3-Aminopropyl-1-butylimidiazoliumchlorid, 3-Aminopropyl-1-methylimidiazoliumbromid, 3-Aminopropyl-1-butylimidiazoliumbromid, 3-Aminoethyl-1-methylimidiazoliumchlorid, 3-Aminoethyl-1-butylimidiazoliumchlorid, 3-Aminoethyl-1-methylimidiazoliumbromid und 3-Aminoethyl-1-butylimidiazoliumbromid.

Für den Fall k = 1 können Polyetheramine der allgemeinen Formel (III) im erfindungsgemäßen Verfahren in Form von Handelsprodukten der Huntsman Corporation wie Jeffamin M-600, Jeffamin M-1000, Jeffamin M-2005 und Jeffamin M-2070 eingesetzt werden.

Wenn k eine ganze Zahl größer 1 ist, können im erfindungsgemäßen Verfahren beispielsweise die folgenden Amine der allgemeinen Formel (III) eingesetzt werden: Polyetherdiamine, Ethylendiamin, Propylendiamin, Butylendiamin, Hexamethylendiamin, MDA, TDA, Diaminobenzol, Diaminocyclohexan, Diaminomethylcyclohexan, TCD-Diamin, 1,4-Bis-(3-aminopropyloxy)butan, Tris-(2-aminoethyl)amin, 1,4-Bis-(3-aminopropyl)piperazin, Bis-(3-aminopropyl)amin und Triethylentetramin.

Für den Fall, dass k eine ganze Zahl größer 1 ist, können Polyetherdiamine der Formel (III) im erfindungsgemäßen Verfahren in Form von Handelsprodukten der Huntsman Corporation wie Jeffamin D-230, Jeffamin D-400, Jeffamin D-2000, Jeffamin D-4000, Jeffamin ED-600, Jeffamin ED-2003 und Jeffamin EDR-148 eingesetzt werden.

Möglich ist es auch, bei der Durchführung des erfindungsgemäßen Verfahrens neben dem Amin der allgemeinen Formel (III) eine weitere Hilfsbase einzusetzen, um den bei der Umsetzung entstehenden Chlorwasserstoff zu binden.

Als solche Hilfsbasen können beispielsweise Trimethylamin, Triethylamin, Tripropylamin, Triisopropylamin, Tributylamin, Pyridin, 1,2-Diazin, 1,3- Diazin, 1,4- Diazin, 1,2,3-Triazin, 1,2,4-Triazin, 1,3,5-Triazin, C₁-C₃₀-Alkylpyrrol, Isoazol, Oxazol, Isothiazol, Thiazol, C₁-C₃₀-Alkylpyrazol, C₁-C₃₀-Alkylimidazol, 1,2,4-Thiadiazol, C₁-C₃₀-Alkyl-1,2,3-triazol, C₁-C₃₀-Alkyl-1,2,4-triazol, Dimethylanilin, Dimethylaminopyridin, Natriumcarbonat, Natriumhydrogencarbonat, Kaliumcarbonat, Kaliumhydrogencarbonat, Natriumhydroxid und Kaliumhydroxid verwendet werden. Wenn Alkyl-substituierte Hilfsbasen eingesetzt werden, sind jene bevorzugt, die C₁-C₆-Alkylketten tragen. Weitere mögliche Substitutionsmuster sind beispielsweise in EP-A- 1 470 136 genannt.

Eine Abtrennung der Hydrochloride der Hilfsbasen kann beispielsweise durch Filtration erfolgen. Alternativ kann eine Abtrennung auch durch eine Phasentrennung bei erhöhter Temperatur erfolgen, wenn sich die Salzschmelze gegebenenfalls in Anwesenheit eines inerten Lösungsmittels vom entsprechenden Produkt abscheidet. Ebenso kann eine Aufarbeitung durch Zugabe von Wasser oder einer Salzlösung und gegebenenfalls einem inerten Lösungsmittel und anschließende Phasentrennung erfolgen. Die eingesetzte Hilfsbase kann dann gegebenenfalls durch Zugabe einer starken Base wieder freigesetzt und gegebenenfalls nach Reinigung wieder recyclisiert werden.

Wenn derartige Hilfsbasen eingesetzt werden, so werden üblicherweise bis zu 3 Äquivalente der Hilfsbase bezogen auf die Aminkomponente der allgemeinen Formel (III) eingesetzt. Bevorzugt werden bis zu 2 und besonders bevorzugt bis zu 1,5 Äquivalente eingesetzt.

Bezogen auf die eingesetzte Verbindung der allgemeinen Formel (III) werden nach dem erfindungsgemäßen Verfahren 0,75 bis 4 Äquivalente des Chlorformiats der allgemeinen Formel (II) eingesetzt. Bevorzugt werden 0,9 bis 3 Äquivalente, besonders bevorzugt 1,0 bis 2,0 Äquivalente eingesetzt.

Das erfindungsgemäße Verfahren wird üblicherweise bei einer Temperatur im Bereich von -10°C bis 100°C durchgeführt. Bevorzugt wird das erfindungsgemäße Verfahren bei einer Temperatur im Bereich von -5°C bis 80°C durchgeführt, besonders bevorzugt im Bereich von 0°C bis 60°C und insbesondere im Bereich von 0°C bis 40°C.

Das erfindungsgemäße Verfahren kann so durchgeführt werden, dass das Amin der allgemeinen Formel (III), gegebenenfalls gelöst in einem inerten Lösungsmittel, vorgelegt wird und anschließend das Chlorformiat der allgemeinen Formel (II), gegebenenfalls ebenfalls gelöst in dem gleichen oder in einem verschiedenen inerten Lösungsmittel, zu dem Amin gegeben wird. In dieser Ausführungsform kann gegebenenfalls noch eine Hilfsbase verwendet werden, die in den vorgenannten Mengen entweder dem vorgelegten Amin der allgemeinen Formel (III) oder dem zugegebenen Chlorformiat der allgemeinen Formel (II) zugegeben worden sein kann. Weiterhin kann die Hilfsbase auch separat kontinuierlich oder in Portionen zudosiert werden.

In einer alternativen Ausführungsform wird das Chlorformiat der allgemeinen Formel (II) vorgelegt und anschließend das Amin der allgemeinen Formel (III) zugegeben, wobei beide Substanzen gegebenenfalls mit dem gleichen oder verschiedenen inerten Lösungsmittel verdünnt sein können. In dieser Ausführungsform kann ebenfalls eine Hilfsbase in den vorgenannten Mengen entweder bei dem zugegebenen Chlorformiat der allgemeinen Formel (II) oder dem vorgelegten Amin der allgemeinen Formel (III) zugegeben sein. Weiterhin kann die Hilfsbase auch separat kontinuierlich oder in Portionen zudosiert werden.

Es sei ausdrücklich erwähnt, dass beide vorgenannten Ausführungsformen auch in Abwesenheit von Lösungsmitteln durchgeführt werden können.

Geeignete Lösungsmittel sind beispielsweise Toluol, Xylole, Chlorbenzol, 1,2-Dichlorbenzol, Tetrahydrofuran, Diethylether, Dioxan, Methyltetrahydrofuran, Cyclopentylmethylether, Dibutylether, 1,2-Dichlorethan, Dichlormethan, Chloroform, Dimethoxyethan, Acetonitril, Cylohexan, Methylcyclohexan, Cyclopentan, Heptan, Hexan, Diethylenglycoldimethylether, Ethylbenzol und Methyl-tert-butylether.

Sofern im erfindungsgemäßen Verfahren als Verbindungen der allgemeinen Formel (III) Amine eingesetzt werden, die als Reste R² und/oder R³ Mono- oder Di(C₁-C₁₈-alkyl)amino-C₁-C₃₀-alkyl-, Mono- oder Di(C₇-C₁₁-aralkyl)amino-C₁-C₃₀-alkyl- oder Mono- oder Di(allyl)amino-C₁-C₃₀-alkylReste aufweisen, so können die tertiären Aminogruppen in diesen Resten im Rahmen des erfindungsgemäßen Verfahrens auch quaternisiert werden. Diese Quaternisierung erfolgt im Anschluss an das erfindungsgemäße Verfahren beispielsweise durch Zugabe von Methyliodid, Methylbromid, Benzylbromid oder Dimethylsulfat.

Die Isolierung der Verbindungen der allgemeinen Formel (I) kann durch Destillation, Kristallisation, Chromatographie oder Gefriertrocknung erfolgen.

Die Verbindungen der allgemeinen Formel (II) können beispielsweise nach einem in DE-A-197 37 329 beschriebenen Verfahren ausgehend von den entsprechenden Alkoholen der allgemeinen Formel (IV) hergestellt werden, 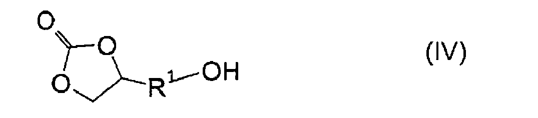 worin R¹ die für die allgemeinen Formel (I) genannte Bedeutung besitzt.

Die nach dem erfindungsgemäßen Verfahren erhaltenen Verbindungen der allgemeinen Formel (I) lassen sich mit funktionellen Gruppen wie z.B. Hydroxygruppen oder primären oder sekundären Aminogruppen umsetzen, die in Biomolekülen, Polymeren oder Substratoberflächen enthalten sind. Hierbei kommt es zur Öffnung des cyclischen Carbonatrings unter kovalenter Anbindung an das Biomolekül, das Polymer oder die Substratoberfläche. Auf diese Weise ist es möglich, die oben definierte Vielzahl von Resten R² und R³ in Biomoleküle oder Polymere einzuführen oder an Substratoberflächen zu binden.

Der Begriff "Biomolekül" soll dabei alle Moleküle umfassen, die aus biologischen Systemen isoliert werden können und/oder mit biologischen Systemen oder Teilen davon in Wechselwirkung treten können. Hierzu zählen vor allem Peptide, Proteine, Proteoglykane, Enzyme, Markierungsstoffe, Antikörper, Rezeptormoleküle, Antigene und Wirkstoffe. Spezielle Beispiele sind Heparin, Gewebeplasminogenaktivator, Streptokinase und Prostaglandine.

Als Polymere sind beispielsweise Polyamine oder Polyole einsetzbar. Beispiele für Polyamine sind Polyvinylamin, Polyallylamin, Polyethylenimine, Chitosan, Polyamid-Epichlorhydrin-Harze, (Erhältlich als "Hercosett®" Produkte), Polyaminostyrol, Peptide oder Proteine, wie Gelatine.

Als Oberflächen, die nach diesem Verfahren behandelt werden können, sind z.B. die Oberflächen von Materialien geeignet, die Amino- und/oder Hydroxygruppen aufweisen, oder Oberflächen, die nach an sich bekannten Verfahren mit Aminosilanen behandelt wurden.

### BEISPIELE

### Beispiel 1 (erfindungsgemäß):

### Herstellung von (2-Oxo-1,3-dioxolan-4-yl)methyl dioctylcarbamat

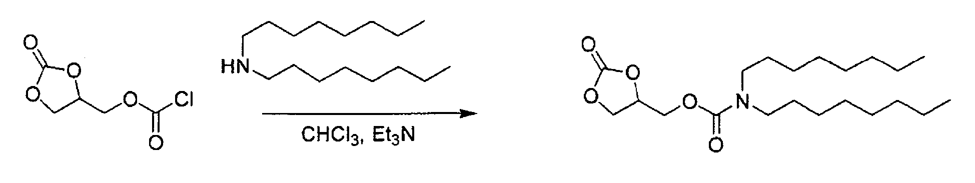

Zu einer Lösung von Dioctylamin (97%ig, 6,936 g) und Triethylamin (2,337 g) in Chloroform (45 ml) wurde bei 0°C unter Rühren eine Lösung aus Glycerolcarbonat-chlorformiat (d.h. (2-Oxo-1,3-dioxolan-4-yl)methyl chlorformiat) (91%ig, 5,562 g) in Chloroform (20 ml) gegeben. Die Lösung wurde eine Stunde bei 0°C und weitere 20 Stunden bei 20°C gerührt. Nach Verdünnung mit je 10 ml THF und 10 mL Diethylether wurde der sich bildende Feststoff abfiltriert und das erhaltene Filtrat mit 10 ml 1%iger HCl-Lösung in Wasser, sowie 10 mL gesättigter Natriumchloridlösung gewaschen. Nach Trocknen der organischen Phase mit 2 g Natriumsulfat wurden die flüchtigen Bestandteile bei 20 mbar und 50°C destillatitv entfernt. Das Produkt wurde als gelbes Öl erhalten (10,912 g, 80% der Theorie).

### Beispiel 2 (erfindungsgemäß):

### Herstellung von (2-Oxo-1,3-dioxolan-4-yl)methyl didodecylcarbamat

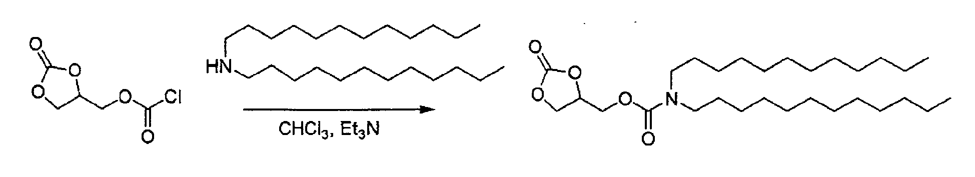

Zu einer Lösung von Didodecylamin (99%ig, 500 mg) und Triethylamin (145 mg) in Chloroform (2 ml) wurde bei 20°C unter Rühren eine Lösung aus Glycerolcarbonat-chlorformiat (91%ig, 300 mg) in Chloroform (1 ml) gegeben. Die Lösung wurde 3 Stunden bei 20°C gerührt. Nach Verdünnung mit je 1 ml THF und 1 mL Diethylether wurde der sich bildende Feststoff abfiltriert und das erhaltene Filtrat mit 10 ml 5%iger HCl-Lösung in Wasser, sowie 10 mL gesättigter Natriumchloridlösung gewaschen. Nach Trocknen der organischen Phase mit 2 g Natriumsulfat wurden die flüchtigen Bestandteile bei 20 mbar und 50°C destillatitv entfernt. Das Produkt wurde als farbloses Öl erhalten (601mg, 86% der Theorie).

### Beispiel 3 (erfindungsgemäß):

### Herstellung von 2-(((2-Oxo-1,3-dioxolan-4-yl)methoxy)carbonylamino)ethansulfonsäure

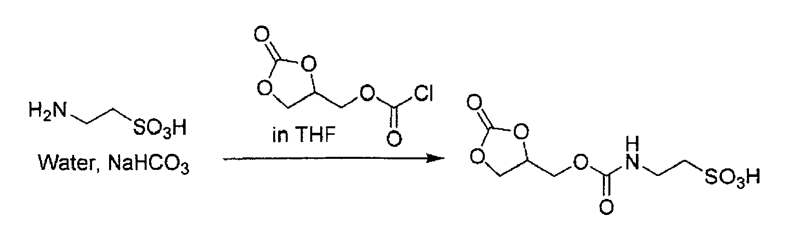

Taurin (99%, 3,88 g) und Natriumhydrogencarbonat (7,81 g) wurden bei 20°C in Wasser (40 ml) suspendiert. Bei 0°C wurde unter Rühren eine Lösung von Glycerolcarbonat-chlorformiat (91%, 6,818 g) in THF (30 mL) zugegeben. Die Lösung wurde eine Stunde bei 0°C und weitere 20 Stunden bei 20°C gerührt. Nach Ansäuern mit 10 ml 5%iger wässriger HCl-Lösung wurde die wässrige Phase dreimal mit je 10 ml Chloroform gewaschen. Die wässrige Phase wurde anschließend gefriergetrocknet. Das Rohprodukt wurde als weisser Feststoff (13,026 g) erhalten.

### Beispiel 4 (erfindungsgemäß):

### Herstellung von (2-Oxo-1,3-dioxolan-4-yl)methyl octadecylcarbamat

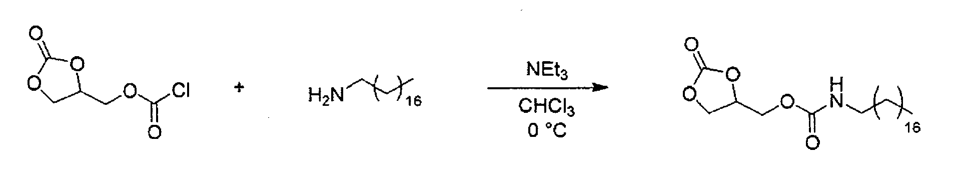

In einem Zweihalskolben mit Tropftrichter und Thermometer wurden Glycerolcarbonat-chlorformiat (91%ig, 9,40 g) in Chlorform (400 ml) gelöst. Bei 0°C wurde eine Suspension von Octadecylamin (14,03 g) und Triethylamin (5,27 g) in Chloroform (70 ml) so zugegeben, dass die Innentemperatur unter 5°C blieb. Nach beendeter Zugabe wurde die Mischung für 32 Stunden bei 20°C gerührt, dann mit Chlorform (200 ml) verdünnt und auf 40°C erhitzt. Bei dieser Temperatur wurde die Mischung einmal mit gesättigter Natriumchloridlösung (400 ml) und einmal mit Wasser (200 ml) gewaschen. Die organische Phase wurde abgetrennt und über 10 g Natriumsulfat getrocknet. Nach Abdestillieren der flüchtigen Bestandteile bei 20 mbar und einer Temperatur von 40°C wurde das erhaltene Rohprodukt aus Chloroform (150 ml) umkristallisiert. Das Produkt wurde als weißes Pulver erhalten (19,49 g, 90,5% d. Th.)

### Beispiel 5 (erfindungsgemäß):

### Herstellung von (2-Oxo-1,3-dioxolan-4-yl)methyl-2-ethylhexanylcarbamat

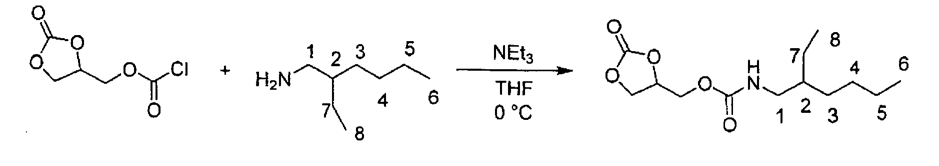

In einem Zweihalskolben mit Tropftrichter und Thermometer wurde Glycerolcarbonat-chlorformiat (91%ig, 4,50 g) in Tetrahydrofuran (60 ml) gelöst. Bei 0°C wurde eine Suspension von Octylamin (2,93 g) und Triethylamin (2,29 g) in Tetrahydrofuran (30 ml) so zugegeben, dass die Innentemperatur unter 5°C blieb. Die Mischung wurde filtriert und die flüchtigen Bestandteile des Filtrats wurden bei 20 mbar und einer Temperatur von 40°C abdestilliert.

### Beispiel 6 (erfindungsgemäß):

### Herstellung von (2-Oxo-1,3-dioxolan-4-yl)methyl-2,4,4-trimethylpent-2-ylcarbamat

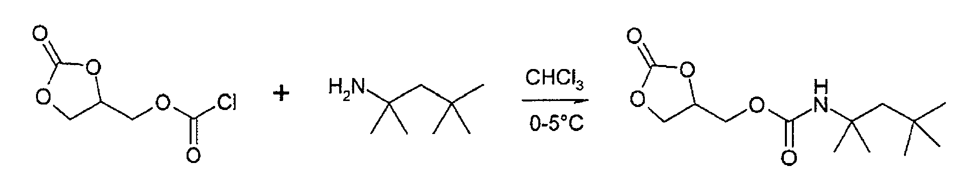

In einem Einhalskolben wurde (2-Oxo-1,3-dioxolan-4-yl)methylchlorformiat (4,784 g) in Chloroform (50 ml) vorgelegt und auf 0 °C gekühlt. Eine Lösung von tert-Octylamin (6,233 g) in Chloroform (40 ml) wurde bei 0 °C langsam zugetropft. Das Gemisch wurde dann 20 Stunden bei 20°C gerührt. Nach Verdünnen mit 70 ml Chloroform wurde die organische Phase dreimal mit je 70 ml Wasser gewaschen. Die organische Phase wurde mit 5 g Na₂SO₄ getrocknet. Nach Abdestillieren der flüchtigen Bestandteile bei 20 mbar und 40°C wurde eine gelbe ölige Flüssigkeit erhalten (6,630 g, 96 % d. Theorie).

## Patentansprüche

1. Verfahren zur Herstellung von Verbindungen der allgemeinen Formel (I), 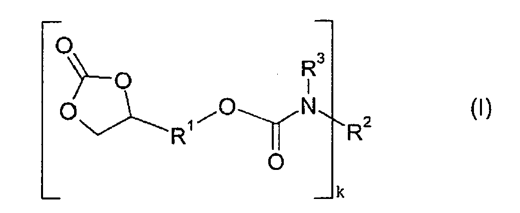 worin
R¹ C₁-C₁₂-Alkylen bedeutet,
k für 1 oder eine ganze Zahl größer als 1 steht,
und wobei, wenn k für 1 steht,
R² und R³ gleich oder verschieden sind und für C₁-C₃₀-Alkyl, C₂-C₃₀-Alkenyl, C₁-C₃₀- Halogenalkyl, C₁-C₃₀-Hydroxyalkyl, C₁-C₆-Alkyloxy-C₁-C₃₀-alkyl, C₁-C₆- Alkylcarbonyloxy-C₁-C₃₀-alkyl, Amino-C₁-C₃₀-alkyl, Mono- oder Di(C₁-C₁₈- alkyl)amino-C₁-C₃₀-alkyl, Mono- oder Di(C₇-C₁₁-aralkyl)amino-C₁-C₃₀-alkyl, Mono- oder Di(allyl)amino-C₁-C₃₀-alkyl, Ammonio-C₁-C₃₀-alkyl, Polyoxyalkylen- C₁-C₃₀-alkyl, Polysiloxanyl-C₁-C₃₀-alkyl, (Meth)acryloyloxy-C₁-C₃₀-alkyl, Sulfono-C₁-C₃₀-alkyl, Phosphono-C₁-C₃₀-alkyl, Di(C₁-C₆-alkyl)phosphono-C₁-C₃₀- alkyl, Phosphonato-C₁-C₃₀-alkyl, Di(C₁-C₆-alkyl)phosphonato-C₁-C₃₀-alkyl, 3- Imidiazolio-C₁-C₃₀-Alkyl oder -[C₂-C₆-Alkylen-O]ₓ-[C₂-C₆-Alkylen-O]_{y}-R, wobei R für Wasserstoff oder C₁-C₆-Alkyl steht, x = 0-50 und y=0-50 ist, entweder x oder y mindestens 1 ist und die beiden Alkylenreste verschieden sind, wenn x und y beide ungleich 0 sind, oder einen Saccharidrest steht, und R³ statt der vorgenannten Bedeutungen alternativ auch für Wasserstoff stehen kann, oder aber R² und R³ unter Einbeziehung des Stickstoffatoms, an das sie gebunden sind, einen cyclischen aliphatischen oder aromatischen Rest bilden, der 4 oder 5 Kohlenstoffatome aufweist, wobei ein oder zwei dieser Kohlenstoffatome zusätzlich durch Heteroatome, ausgewählt aus Stickstoff, Schwefel und Sauerstoff, ersetzt sein können, oder aber
R³ für Wasserstoff steht und gleichzeitig
R² für einen Fluoresceinyl-Rest oder einen über eine C₁-C₃₀-Alkylengruppe gebundenen Pyren-, Fluorescein-, Cumarin-, 4,4'-Bisstyrylbiphenyl-, Pyrazolin-, Hydrochinolon-, Benzoxazol-, Benzisoxazol-, Benzimidazol- oder Flavonsäure- Rest steht,
und wobei, wenn k für eine ganze Zahl größer als 1 steht,
R³ die gleichen Bedeutungen wie für k= besitzt und
R² für einen k-valenten aliphatischen, cycloaliphatischen, aromatischen oder araliphatischen Kohlenwasserstoff-Rest steht, der gegebenenfalls ein oder mehrere Heteroatome enthält,
durch Umsetzung von Verbindungen der allgemeinen Formel (II) 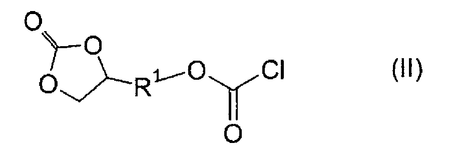 worin R¹ die für die allgemeine Formel (I) genannte Bedeutung hat,
mit einer Verbindung der allgemeinen Formel (III), 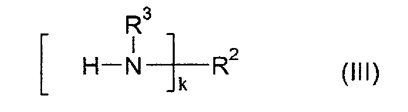 worin k und ebenso R² und R³ in Abhängigkeit von k die für die allgemeine Formel (I) beschriebenen Bedeutungen haben, oder einem Salz davon.

2. Verfahren nach Anspruch 1, wobei Verbindungen der allgemeinen Formel (II) eingesetzt werden, worin R¹ einen C₁-C₆-Alkylen-Rest, bevorzugt einen Methylen-, Ethylen-, n-Propylen, n-Butylen- oder n-Hexamethylenrest darstellt.

3. Verfahren nach Anspruch 1 oder 2, wobei die Verbindungen der allgemeinen Formel (III) in Form der jeweiligen Ammoniumsalze der allgemeinen Formel (IIIA) eingesetzt werden, 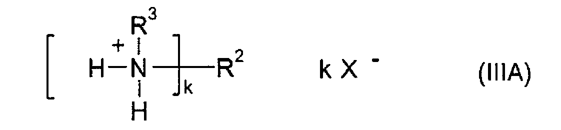 wobei R² und R³ jeweils die im Zusammenhang mit der Formel (III) genannten Bedeutungen besitzen und X für ein Halogenid, bevorzugt Chlor, Brom oder Iod, oder Methylsulfat steht.

4. Verfahren nach einem oder mehreren der Ansprüche 1 bis 3, wobei Verbindungen der allgemeinen Formel (III) eingesetzt werden, bei denen
k gleich 1 ist und
R² und R³ gleich oder verschieden sind und für C₁-C₃₀-Alkyl, C₁-C₆-Alkyloxy-C₁-C₃₀-alkyl, C₁-C₆-Alkylcarbonyloxy-C₁-C₃₀-alkyl, Amino-C₁-C₃₀-alkyl, Mono- oder Di(C₁-C₆- alkyl)amino-C₁-C₃₀-alkyl, Mono- oder Di(C₇-C₁₁-aralkyl)amino-C₁-C₃₀-alkyl, Mono- oder Di(allyl)amino-C₁-C₃₀-alkyl, Ammonio-C₁-C₃₄-alkyl, Polyoxyalkylen- C₁-C₃₀-alkyl, Polysiloxanyl-C₁-C₃₀-alkyl, (Meth)acryloyloxy-C₁-C₃₀-alkyl, 3- Imidazolio-C₁-C₃₀-Alkyl oder [C₂-C₆-Alkylen-O]ₓ-[C₂-C₆-Alkylen-O]_{y}-C₁-C₆- Alkyl, wobei x = 0-50 und y = 0-40 sind, entweder x oder y mindestens 1 ist und die beiden Alkylenreste verschieden sind, wenn x und y beide ungleich 0 sind, stehen oder
R³ auch für Wasserstoff stehen kann.

5. Verfahren nach einem oder mehreren der Ansprüche 1 bis 4, wobei Verbindungen der allgemeinen Formel (III) eingesetzt werden, bei denen
k gleich 1 ist
R² für C₁-C₁₈-Alkyl, C₁-C₆-Alkyloxy-C₁-C₁₈-alkyl, C₁-C₆-Alkylcarbonyloxy-C₁-C₁₈- alkyl, Amino-C₁-C₁₈-alkyl, Mono- oder Di(C₁-C₆-alkyl)amino-C₁-C₁₈-alkyl, Mono- oder Di(C₇-C₁₁-aralkyl)amino-C₁-C₁₈-alkyl, Mono- oder Di(allyl)ammo-C₁-C₁₈- alkyl, Ammonio-C₁-C₁₈-alkyl, Polyoxyalkylen-C₁-C₁₈-alkyl, Polysiloxanyl-C₁-C₁₈- alkyl, (Meth)acryloyloxy-C₁-C₁₈-alkyl, 3-Imidazolio-C₁-C₃₀-Alkyl oder [C₂-C₆- Alkylen-O]ₓ-[C₂-C₆-Alkylen-O]_{y}-C₁-C₆-Alkyl, wobei die beiden Alkylenreste verschieden sind und x = 1-50 und y = 1-40, steht und
R³ Wasserstoff bedeutet.

6. Verfahren nach einem oder mehreren der Ansprüche 1 bis 5, wobei Verbindungen der allgemeinen Formel (III) eingesetzt werden, bei denen
k gleich 1 ist und
R² und R³ gleich oder verschieden sind und für C₁-C₁₈-Alkyl, Amino-C₁-C₁₈-alkyl, Mono- oder Di(C₁-C₆-alkyl)amino-C₁-C₁₈-alkyl, Mono- oder Di(C₇-C₁₁-aralkyl)amino-C₁- C₁₈-alkyl, Mono- oder Di(allyl)amino-C₁-C₁₈-alkyl, Ammonio-C₁-C₁₈-alkyl, Polyoxyalkylen-C₁-C₁₈-alkyl, Polysiloxanyl-C₁-C₁₈-alkyl, 3-Imidazolio-C₁-C₃₀- Alkyl oder [C₂-C₆-Alkylen-O]ₓ-[C₂-C₆-Alkylen-O]_{y}-C₁-C₆-Alkyl, wobei die beiden Alkylenreste verschieden sind und x = 2-40 und y =2-20, stehen.

7. Verfahren nach einem oder mehreren der Ansprüche 1 bis 3, wobei Verbindungen der allgemeinen Formel (III) eingesetzt werden, bei denen
k gleich 1 ist,
R³ Wasserstoff bedeutet und
R² für -(CH₂)ₙ-CH₃,
-(CH₂)ₙ-CH((CH₂)ₘ-CH₃)-(CH₂)ₒ-CH₃, wobei n+m+o maximal 27 ergeben,
-(CH₂)ₙ-(CF₂)ₚ-CF₃,
-(CH₂)ₙ-[O-Si(-O-Si(CH₃)₃)₃],
-(CH₂)ₙ-(O-CH₂-CH₂)_{q}-O-R⁴-H,
-R⁴-OH,
-R⁴-NR⁵₂,
-R⁴-NR⁵₃⁺ Y',
-R⁴-OC(=O)-CR⁶=CH₂,
-R⁴-SO₃H,
-R⁴-PO₃H₂,
-R⁴-OPO₃H₂
oder einen Saccharidrest steht,
worin
R⁴ für C₁-C₆-Alkylen,
R⁵ für Wasserstoff, C₁-C₁₈ Alkyl, C₂-C₁₈ Alkenyl, bevorzugt Allyl, oder Benzyl,
R⁶ für Wasserstoff oder Methyl und
Y für ein Äquivalent eines Anions, bevorzugt Chlorid, Bromid, Sulfat, Hydrogensulfat, Methosulfat oder Nitrat steht,
n, m,o unabhängig voneinander eine ganze Zahl von 0 bis 17 sind,
p eine ganze Zahl von 1 bis 12 ist und
q für eine ganze Zahl von 1 bis 100, bevorzugt 2 bis 50 steht.

8. Verfahren nach einem oder mehreren der Ansprüche 1 bis 3, wobei Verbindungen der allgemeinen Formel (III) eingesetzt, bei denen
k eine ganze Zahl größer 1, insbesondere 2 oder 3 ist,
R² für einen k-valenten, insbesondere di- oder trivalenten, aliphatischen, cycloaliphatischen, aromatischen oder araliphatischen Kohlenwasserstoff-Rest steht, der gegebenenfalls ein oder mehrere Heteroatome enthält, und
R³ für Wasserstoff, C₁-C₃₀-Alkyl, C₁-C₆-Alkyloxy-C₁-C₃₀-alkyl, C₁-C₆- Alkylcarbonyloxy-C₁-C₃₀-alkyl, Amino-C₁-C₃₀-alkyl, Mono- oder Di(C₁-C₆-alkyl)- amino-C₁-C₃₀-alkyl, Mono- oder Di(C₇-C₁₁-aralkyl)amino-C₁-C₃₀-alkyl, Mono- oder Di(allyl)amino-C₁-C₃₀-alkyl, Ammonio-C₁-C₃₀-alkyl, Polyoxyalkylen-C₁-C₃₀- alkyl, Polysiloxanyl-C₁-C₃₀-alkyl, (Meth)acryloyloxy-C₁-C₃₀-alkyl, 3-Imidazolio- C₁-C₃₀-Alkyl oder [C₂-C₅-Alkylen-O]ₓ-[C₂-C₆-Alkylen-O]_{y}-C₂-C₆-Alkyl, wobei x = 0-50 und y= 0-50 sind, aber entweder x oder y mindestens 1 ist und die beiden Alkylenreste verschieden sind, wenn x und x ungleich 0 sind, steht.

9. Verfahren nach einem oder mehreren der Ansprüche 1 bis 3 oder 8, wobei Verbindungen der allgemeinen Formel (III) eingesetzt, bei denen
k 2 oder 3 ist,
R² für einen di- oder trivalenten, aliphatischen, cycloaliphatischen, aromatischen oder araliphatischen Kohlenwasserstoff-Rest steht, der gegebenenfalls ausschließlich ein oder mehrere Sauerstoffatome als Heteroatome enthält, und
R³ für Wasserstoff, C₁-C₁₈-Alkyl, C₁-C₆-Alk-yloxy-C₁-C₁₈-alkyl, C₁-C₆- Alkylcarbonyloxy-C₁-C₁₈-alkyl, Amino-C₁-C₁₈-alkyl, Mono- oder Di(C₁-C₆-alkyl)- amino-C₁-C₁₈-alkyl, Ammonio-C₁-C₁₈-alkyl, Polyoxyalkylen-C₁-C₁₈-alkyl, Polysiloxanyl-C₁-C₁₈-alkyl, (Meth)acryloyloxy-C₁-C₁₈-alk-yl, 3-Imidazolio-C₁-C₃₀- Alkyl oder [C₂-C₆-Alkylen-O]ₓ-[C₁-C₆-Alkylen-O]ₓ-C₂-C₆-Alkyl, wobei x = 1-50 und y = 1-50 und die beiden Alkylenreste verschieden sind, steht.

10. Verfahren nach einem oder mehreren der Ansprüche 1-3 und 8-9, wobei Verbindungen der allgemeinen Formel (III) eingesetzt, bei denen
k 2 ist,
R² eine -[(C₂-C₆-Alkylen-O-)ₓ-(C₂-C₆-Alkylen)]- Gruppe, worin die beiden Alkylengruppen gleich oder verschieden sind und x = 1-50 ist, bevorzugt x = 1-40, oder eine -[(C₂-C₆-Alkylen-O-)ₓ-(C₂-C₆-Alkylcn-O-)y-(C₂-C₆-Alkylcn-O-)_{z}-C₂-C₆-Alkylen]- Gruppe darstellt, wobei die drei Alkyleneinheiten verschieden sind bzw. die erste mit der dritten identisch sein kann, und wobei x = 0-50, inbesondere x = 1-40, y = 1-50, insbesondere y = 1-40, und z = 1-50, insbesondere z = 1-40 und
R³ Wasserstoff oder C₁-C₃₀-Alkyl bedeutet.

11. Verfahren nach einem oder mehreren der Ansprüche 1 bis 3, wobei für den Fall, dass k = 1 ist, als Verbindungen der allgemeinen Formel (III) Polyetheramine, n-Octylamin, tert-Octylamin, n-Hexylamin, 2-Ethylhexylamin, Taurin, Dihexylamin, Dodecylamin, Octadecylamin, Methyloctadecylamin, Dioctylamin, N,N-Dimethylpropylendiamin, N,N-Dimethylethylendiamin, 3-Aminopropyltrimethylammoniumiodid, 3-Aminopropyltrimethylammoniumbromid, 3-Aminopropyltrimethylammoniumchlorid, 3-Aminoethyltrimethylammoniumiodid, 3-Aminoethyltrimethylammoniumbromid, 3-Aminoethyltrimethylammoniumchlorid, 3-Aminopropyldimethyloctylammoniumchlorid, 3-Aminopropyldimethyloctylammoniumbromid, 3-(Trimethylsilyl)-propylamin, 3-(Tris(trimethylsilyloxy)silyl)-propylamin, 3-(Trimethylsilyl)-ethylamin, 3-(Tris(trimethylsilyloxy)silyl)-ethylamin, 3-Aminopropylimidazol, 2-Aminoethylimidazol, 4-Aminobutylimidazol, 3-Aminopropyl-1-methylimidiazolium-methylsulfat, 3-Aminopropyl-1-butylimidiazoliummethylsulfat, 3-Aminoethyl-1-methyl-imidiazoliummethylsulfat, 3-Aminoethyl-1-butylimidiazoliummethylsulfat, 3-Aminopropyl-1-methylimidiazoliumchlorid, 3-Aminopropyl-1-butylimidiazoliumchlorid, 3-Aminopropyl-1-methylimidiazoliumbromid, 3-Aminopropyl-1-butylimidiazoliumbromid, 3-Aminoethyl-1-methylimidiazoliumchlorid, 3-Aminoethyl-1-butylimidiazoliumchlorid, 3-Aminoethyl-1-methylimidiazoliumbromid oder 3-Aminoethyl-1-butylimidiazoliumbromid eingesetzt werden.

12. Verfahren nach einem oder mehreren der Ansprüche 1 bis 3, wobei als Verbindungen der allgemeinen Formel (III) für den Fall, dass k eine ganze Zahl größer 1 ist, Polyetherdiamine, Ethylendiamin, Propylendiamin, Butylendiamin, Hexamethylendiamin, MDA, TDA, Diaminobenzol, Diaminocyclohexan, Diaminomethylcyclohexan, TCD-Diamin, 1,4-Bis-(3-aminopropyloxy)butan, Tris-(2-aminoethyl)amin, 1,4-Bis-(3-aminopropyl)piperazin, Bis-(3-aminopropyl)amin oder Triethylentetramin eingesetzt werden.

13. Verfahren nach einem oder mehreren der Ansprüche 1 bis 12, wobei zusätzlich eine Hilfsbase eingesetzt wird, bevorzugt Trimethylamin, Triethylamin, Tripropylamin, Triisopropylamin, Tributylamin, Pyridin, 1,2-Diazin, 1,3- Diazin, 1,4- Diazin, 1,2,3-Triazin, 1,2,4-Triazin, 1,3,5-Triazin, C₁-C₃₀-Alkylpyrrol, Isoazol, Oxazol, Isothiazol, Thiazol, C₁-C₃₀-Alkylpyrazol, C₁-C₃₀-Alkylimidazol, 1,2,4-Thiadiazol, C₁-C₃₀-Alkyl-1,2,3-triazol, C₁-C₃₀-Alkyl-1,2,4-triazol, Dimethylanilin, Dimethylaminopyridin, Natriumcarbonat, Natriumhydrogencarbonat, Kaliumcarbonat, Kaliumhydrogencarbonat, Natriumhydroxid oder Kaliumhydroxid.

14. Verfahren nach einem oder mehreren der Ansprüche 1 bis 13, wobei bezogen auf die eingesetzte Verbindung der allgemeinen Formel (III) 0,75 bis 4 Äquivalente des Chlorformiats der allgemeinen Formel (II) eingesetzt werden, bevorzugt 0,9 bis 3 Äquivalente, besonders bevorzugt 1,0 bis 2,0 Äquivalente.

15. Verfahren nach einem oder mehreren der Ansprüche 1 bis 14, wobei das Verfahren bei einer Temperatur im Bereich von -10°C bis 100°C durchgeführt wird, bevorzugt bei einer Temperatur im Bereich von -5°C bis 80°C, besonders bevorzugt im Bereich von 0°C bis 60°C und insbesondere im Bereich von 0°C bis 40°C.

## Claims

1. Process for preparing compounds of the general formula (I), 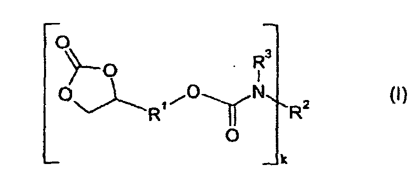 in which
R¹ is C₁-C₁₂-alkylene,
k is 1 or an integer greater than 1,
and where, when k is 1,
R² and R³ are the same or different and are each C₁-C₃₀-alkyl, C₂-C₃₀-alkenyl, C₁-C₃₀-haloalkyl, C₁-C₃₀-hydroxyalkyl, C₁-C₆-alkyloxy-C₁-C₃₀-alkyl, C₁-C₆-alkylcarbonyloxy-C₁-C₃₀-alkyl, amino-C₁-C₃₀-alkyl, mono- or di(C₁-C₁₈-alkyl)amino-C₁-C₃₀-alkyl, mono- or di(C₇-C₁₁-aralkyl)amino- C₁-C₃₀-alkyl, mono- or di(allyl)amino-C₁-C₃₀-alkyl, ammonio-C₁-C₃₀-alkyl, polyoxyalkylene-C₁-C₃₀-alkyl, polysiloxanyl-C₁-C₃₀-alkyl, (meth)acryloyloxy-C₁-C₃₀-alkyl, sulphono-C₁-C₃₀-alkyl, phosphono- C₁-C₃₀-alkyl, di(C₁-C₆-alkyl)phosphono-C₁-C₃₀-alkyl, phosphonato-C₁-C₃₀- alkyl, di(C₁-C₆-alkyl)phosphonato-C₁-C₃₀-alkyl, 3-imidazolino-C₁-C₃₀- alkyl or -[C₂-C₆-alkylene-O]ₓ-[C₂-C₆-alkylene-O]y-R, where R is hydrogen or C₁-C₆-alkyl, x = 0-50 and y = 0-50, either x or y is at least 1 and the two alkylene radicals are different when neither x nor y is 0, or a saccharide radical, and
R³, instead of the above definitions, may alternatively also be hydrogen, or else
R² and R³, including the nitrogen atom to which they are bonded, form a cyclic aliphatic or aromatic radical which has 4 or 5 carbon atoms, where one or two of these carbon atoms may additionally be replaced by heteroatoms selected from nitrogen, sulphur and oxygen, or else
R³ is hydrogen and, at the same time,
R² is a fluoresceinyl radical or a C₁-C₃₀-alkylene-bonded pyrene, fluorescein, coumarin, 4,4'-bisstyrylbiphenyl, pyrazoline, hydroquinolone, benzoxazole, benzisoxazole, benzimidazole or flavonic acid radical,
and where, when k is an integer greater than 1,
R³ has the same meanings as when k = 1 and
R² is a k-valent aliphatic, cycloaliphatic, aromatic or araliphatic hydrocarbon radical which optionally contains one or more heteroatoms,
by reaction of compounds of the general formula (II) 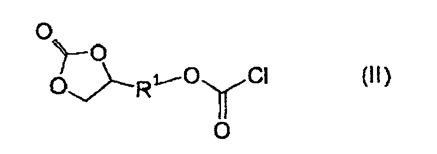 in which R¹ is as defined for the general formula (I) with a compound of the general formula (III) 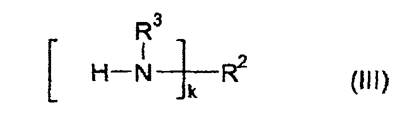 in which k and likewise R² and R³, depending on k, are each defined as described for the general formula (I), or a salt thereof.

2. Process according to Claim 1, wherein compounds of the general formula (II) in which R¹ is a C₁-C₆-alkylene radical, preferably a methylene, ethylene, n-propylene, n-butylene or n-hexamethylene radical, are used.

3. Process according to Claim 1 or 2, wherein the compounds of the general formula (III) are used in the form of the particular ammonium salts of the general formula (IIIA) 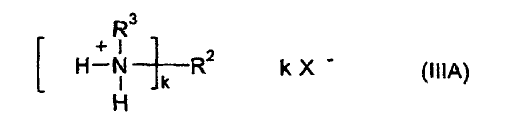 where R² and R³ are each as defined in connection with the formula (III), and X is a halide, preferably chlorine, bromine or iodine, or methylsulphate.

4. Process according to one or more of Claims 1 to 3, wherein compounds of the general formula (III) in which
k is 1 and
R² and R³ are the same or different and are each C₁-C₃₀-alkyl, C₁-C₆-alkyloxy- C₁-C₃₀-alkyl, C₁-C₆-alkylcarbonyloxy-C₁-C₃₀-alkyl, amino-C₁-C₃₀-alkyl, mono- or di(C₁-C₆-alkyl)amino-C₁-C₃₀-alkyl, mono- or di(C₇-C₁₁- aralkyl)amino-C₁-C₃₀-alkyl, mono- or di(allyl)amino-C₁-C₃₀-alkyl, ammonio-C₁-C₃₀-alkyl, polyoxyalkylene-C₁-C₃₀-alkyl, polysiloxanyl- C₁-C₃₀-alkyl, (meth)acryloyloxy-C₁-C₃₀-alkyl, 3-imidazolino-C₁-C₃₀-alkyl or [C₂-C₆-alkylene-O]ₓ-[C₂-C₆-alkylene-O]_{y}-C₁-C₆-alkyl, where x = 0-50 and y = 0-40, either x or y is at least 1 and the two alkylene radicals are different when neither x nor y is 0, or
R³ may also be hydrogen,
are used.

5. Process according to one or more of Claims 1 to 4, wherein compounds of the general formula (III) in which
k is 1,
R² is C₁-C₁₈-alk_{Y}l, C₁-C₆-alkyloxy-C₁-C₁₈-alkyl, C₁-C₆-alkylcarbonyloxy- C₁-C₁₈-alkyl, amino-C₁-CW₁₈-alkyl, mono- or di(C₁-C₆-alkyl)amino-C₁-C₁₈- alkyl, mono- or di(C₇-C₁₁-aralkyl)amino-C₁-C₁₈-alkyl, mono- or di(allyl)- amino-C₁-C₁₈-alkyl, ammonio-C₁-C₁₈-alkyl, polyoxyalkylene-C₁-C₁₈-alkyl, polysiloxanyl-C₁-C₁₈-alkyl, (meth)acryloyloxy-C₁-C₁₈-alkyl, 3-imidazo- lino-C₁-C₃₀-alkyl or [C₂-C₆-alkylene-O]ₓ-[C₂-C₆-alkylene-O]_{y}-C₁-C₆-alkyl, where the two alkylene radicals are different and x = 1-50 and y = 1-40, and
R³ is hydrogen,
are used.

6. Process according to one or more of Claims 1 to 5, wherein compounds of the general formula (III) in which
k is 1 and
R² and R³ are the same or different and are each C₁-C₁₈-alkyl, amino-C₁-C₁₈-alkyl, mono- or di(C₁-C₆-alkyl)amino-C₁-C₁₈-alkyl, mono- or di(C₇-C₁₁- aralkyl)amino-C₁-C₁₈-alkyl, mono- or di(allyl)amino-C₁-C₁₈-alkyl, ammonio-C₁-C₁₈-alkyl, polyoxyalkylene-C₁-C₁₈-alkyl, polysiloxanyl- C₁-C₁₈-alkyl, 3-imidazolino-C₁-C₃₀-alkyl or [C₂-C₆-alkylene-O]ₓ-[C₂-C₆- alkylene-O]_{y}-C₁-C₆-alkyl, where the two alkylene radicals are different and x = 2-40 and y = 2-20,
are used.

7. Process according to one or more of Claims 1 to 3, wherein compounds of the general formula (III) in which
k is 1,
R³ is hydrogen and
R² is -(CH₂)ₙ-CH₃,
-(CH₂)ₙ-CH((CH₂)ₘ-CH₃)-(CH₂)ₒ-CH₃, where n+rn+o is a maximum of 27,
-(CH₂)ₙ-(CF₂)ₚ-CF₃,
-(CH₂)ₙ-[O-Si(-O-Si(CH₃)₃)₃],
-(CH₂)ₙ-(O-CH₂-CH₂)_{q}-O-R⁴-H,
-R⁴-OH
-R⁴-NR⁵₂,
-R⁴-NR⁵₃⁺Y⁻,
-R⁴-OC(=O)-CR⁶=CH₂,
-R⁴-SO₃H,
-R⁴-PO₃H₂,
-R⁴-OPO₃H₂
or a saccharide radical,
in which
R⁴ is C₁-C₆-alkylene,
R⁵ is hydrogen, C₁-C₁₈-alkyl, C₂-C₁₈-alkenyl, preferably allyl, or benzyl,
R⁶ is hydrogen or methyl and
Y is one equivalent of an anion, preferably chloride, bromide, sulphate, hydrogensulphate, methosulphate or nitrate,
n,m,o are each independently an integer from 0 to 17,
p is an integer from 1 to 12 and
q is an integer from 1 to 100, preferably 2 to 50,
are used.

8. Process according to one or more of Claims 1 to 3, wherein compounds of the general formula (III) in which
k is an integer greater than 1, especially 2 or 3,
R² is a k-valent, especially di- or trivalent, aliphatic, cycloaliphatic, aromatic or araliphatic hydrocarbon radical which optionally contains one or more heteroatoms, and
R³ is hydrogen, C₁-C₃₀-alkyl, C₁-C₆-alkyloxy-C₁-C₃₀-alkyl, C₁-C₆- alkylcarbonyloxy-C₁-C₃₀-alkyl, amino-C₁-C₃₀-alkyl, mono- or di(C₁-C₆- alkyl)amino-C₁-C₃₀-alkyl, mono- or di(C₇-C₁₁-aralkyl)amino-C₁-C₃₀-alkyl, mono- or di(allyl)amino-C₁-C₃₀-alkyl, ammonio-C₁-C₃₀-alkyl, polyoxyalkylene-C₁-C₃₀-alkyl, polysiloxanyl-C₁-C₃₀-alkyl, (meth)acryloyloxy-C₁-C₃₀-alkyl, 3-imidazolino-C₁-C₃₀-alkyl or [C₂-C₆- alkylene-O]ₓ-[C₂-C₆-alkylene-O]_{y}-C₂-C₆-alkyl, where x = 0-50 and y = 0- 50, but either x or y is at least 1 and the two alkylene radicals are different when x and y are not 0,
are used.

9. Process according to one or more of Claims 1 to 3 or 8, wherein compounds of the general formula (III) in which
k is 2 or 3,
R² is a di- or trivalent, aliphatic, cycloaliphatic, aromatic or araliphatic hydrocarbon radical which optionally contains exclusively one or more oxygen atoms as heteroatoms, and
R³ is hydrogen, C₁-C₁₈-alkyl, C₁-C₆-alkyloxy-C₁-C₁₈-alkyl, C₁-C₆- alkylcarbonyloxy-C₁-C₁₈-alkyl, amino-C₁-C₁₈-alkyl, mono- or di(C₁-C₆- alkyl)amino-C₁-C₁₈-alkyl, ammonio-C₁-C₁₈-alkyl, polyoxyalkylene-C₁-C₁₈- alkyl, polysiloxanyl-C₁-C₁₈-alkyl, (meth)acryloyloxy-C₁-C₁₈-alkyl, 3- imidazolino-C₁-C₃₀-alkyl or [C₂-C₆-alkylene-O]ₓ-[C₂₋C₆-alkylene-O]_{y}- C₂-C₆-alkyl, where x = 1-50 and y = 1-50 and the two alkylene radicals are different,
are used.

10. Process according to one or more of Claims 1-3 and 8-9, wherein compounds of the general formula (III) in which
k is 2,
R² is a -[(C₂-C₆-alkylene-O-)x-(C₂-C₆-alkylene)]- group in which the two alkylene groups are the same or different and x = 1-50, preferably x = 1-40, or a -[(C₂-C₆-alkylene-O-)ₓ-(C₂-C₆-alkylene-O-)_{y}-(C₂-C₆-alkylene-O-)₂-C₂-C₆- alkylene]- group, where the three alkylene units are different or the first may be identical to the third, and where x = 0-50, especially x = 1-40, y = 1-50, especially y = 1-40, and z = 1-50, especially z = 1-40, and
R³ is hydrogen or C₁-C₃₀-alkyl,
are used.

11. Process according to one or more of Claims 1 to 3, wherein, in the case that k = 1, the compounds of the general formula (III) used are polyetheramines, n-octylamine, tert-octylamine, n-hexylamine, 2-ethylhexylamine, taurine, dihexylamine, dodecylamine, octadecylamine, methyloctadecylamine, dioctylamine, N,N-dimethylpropylenediamine, N,N-dimethylethylenediamine, 3-aminopropyltrimethylammonium iodide, 3-aminopropyltrimethylammonium bromide, 3-aminopropyltrimethylammonium chloride, 3-aminoethyltrimethylammonium iodide, 3-aminoethyltrimethylammonium bromide, 3-aminoethyltrimethylammonium chloride, 3-aminopropyldimethyloctylammonium chloride, 3-aminopropyldimethyloctylammonium bromide, 3-(trimethylsilyl)propylamine, 3-(tris(trimethylsilyloxy)silyl)propylamine, 3-(trimethylsilyl)ethylamine, 3-(tris(trimethylsilyloxy)silyl)ethylamine, 3-aminopropylimidazole, 2-aminoethylimidazole, 4-aminobutylimidazole, 3-aminopropyl-1-methylimidazolium methylsulphate, 3-aminopropyl-1-butylimidazolium methylsulphate, 3-aminoethyl-1-methylimidazolium methylsulphate, 3-aminoethyl-1-butylimidazolium methylsulphate, 3-aminopropyl-1-methylimidazolium chloride, 3-aminopropyl-1-butylimidazolium chloride, 3-aminopropyl-1-methylimidazolium bromide, 3-aminopropyl-1-butylimidazolium bromide, 3-aminoethyl-1-methylimidazolium chloride, 3-aminoethyl-1-butylimidazolium chloride, 3-aminoethyl-1-methylimidazolium bromide or 3-aminoethyl-1-butylimidazolium bromide.

12. Process according to one or more of Claims 1 to 3, wherein the compounds of the general formula (III) used, in the case that k is an integer greater than 1, are polyetherdiamines, ethylenediamine, propylenediamine, butylenediamine, hexamethylenediamine, MDA, TDA, diaminobenzene, diaminocyclohexane, diaminomethylcyclohexane, TCD diamine, 1,4-bis(3-aminopropyloxy)butane, tris(2-aminoethyl)amine, 1,4-bis(3-aminopropyl)piperazine, bis(3-aminopropyl)amine or triethylenetetramine.

13. Process according to one or more of Claims 1 to 12, wherein an auxiliary base is additionally used, preferably trimethylamine, triethylamine, tripropylamine, triisopropylamine, tributylamine, pyridine, 1,2-diazine, 1,3-diazine, 1,4-diazine 1,2,3-triazine, 1,2,4-triazine, 1,3,5-triazine, C₁-C₃₀-alkylpyrrole, isoazole, oxazole, isothiazole, thiazole, C₁-C₃₀-alkylpyrazole, C₁-C₃₀-alkylimidazole, 1,2,4-thiadiazole, C₁-C₃₀-alykl-1,2,3-triazole, C₁-C₃₀-alkyl-1,2,4-triazole, dimethylaniline, dimethylaminopyridine, sodium carbonate, sodium hydrogencarbonate, potassium carbonate, potassium hydrogencarbonate, sodium hydroxide or potassium hydroxide.

14. Process according to one or more of Claims 1 to 13, wherein, based on the compound of the general formula (III) used, 0.75 to 4 equivalents of the chloroformate of the general formula (II) are used, preferably 0.9 to 3 equivalents, more preferably 1.0 to 2.0 equivalents.

15. Process according to one or more of Claims 1 to 14, which is performed at a temperature in the range from -10°C to 100°C, preferably at a temperature in the range from -5°C to 80°C, more preferably in the range from 0°C to 60°C and especially in the range from 0°C to 40°C.

## Revendications

1. Procédé pour la préparation de composés de formule générale (I), 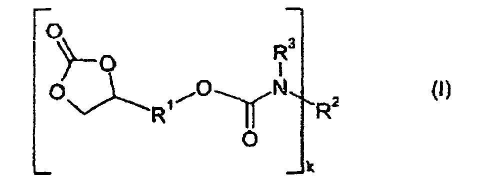 où
R¹ signifie (C₁-C₁₂)-alkylène,
k vaut 1 ou un nombre entier supérieur à 1,
et lorsque k vaut 1,
R² et R³ sont identiques ou différents et représentent C₁-C₃₀-alkyle, C₂-C₃₀-alcényle, C₁-C₃₀- halogénoalkyle, C₁-C₃₀-hydroxyalkyle, C₁-C₆- alkyloxy-C₁-C₃₀-alkyle, C₁-C₆-alkylcarbonyloxy-C₁- C₃₀-alkyle, amino-C₁-C₃₀-alkyle, mono(C₁-C₁₈- alkyl)amino-C₁-C₃₀-alkyle ou di(C₁-C₁₈-alkyl)amino- C₁-C₃₀-alkyle, mono(C₇-C₁₁-aralkyl)amino-C₁-C₃₀- alkyle ou di(C₇-C₁₁-aralkyl)amino-C₁-C₃₀-alkyle, mono(allyl)amino-C₁-C₃₀-alkyle ou di(allyl)amino-C₁- C₃₀-alkyle, ammonio-C₁-C₃₀-alkyle, polyoxyalkylène- C₁-C₃₀-alkyle, polysiloxanyl-C₁-C₃₀-alkyle, (méth) acryloyloxy-C₁-C₃₀-alkyle, sulfono-C₁-C₃₀- alkyle, phosphono-C₁-C₃₀-alkyle, di(C₁-C₆- alkyl) phosphono-C₁-C₃₀-alkyle, phosphonato-C₁-C₃₀- alkyle, di(C₁-C₆-alkyl)phosphonato-C₁-C₃₀-alkyle, 3- imidiazolio-C₁-C₃₀-alkyle ou -[C₂-C₆-alkylène-O]ₓ-
R³ [C₂-C₆-alkylène-O]_{y}-R, où R représente hydrogène ou C₁-C₆-alkyle, x = 0-50 et y = 0-50, soit x soit y vaut au moins 1 et les deux radicaux alkylène sont différents lorsque x et y sont tous les deux différents de zéro, ou un radical saccharide, et peut également représenter, à la place des significations susmentionnées, en variante, hydrogène, ou encore
R² et R³ en incluant l'atome d'azote auquel ils sont liés, forment un radical cyclique aliphatique ou aromatique, qui comprend 4 ou 5 atomes de carbone, où un ou deux de ces atomes de carbone peuvent en outre être remplacés par des hétéroatomes, choisis parmi l'azote, le soufre et l'oxygène ou encore
R³ représente hydrogène et simultanément
R² représente un radical fluorescéinyle ou un radical pyrène, fluorescéine, coumarine, 4,4'- bistyrylbiphényle, pyrazoline, hydroquinolone, benzoxazole, benzisoxazole, benzimidazole ou acide flavonique lié via un groupe C₁-C₃₀-alkylène,
et où, lorsque k vaut un nombre entier supérieur à 1,
R³ présente les mêmes significations que pour k = 1 et
R² représente un radical hydrocarboné de valence k, aliphatique, cycloaliphatique, aromatique ou araliphatique, qui contient le cas échéant un ou plusieurs hétéroatomes,
par transformation de composés de formule générale (II) 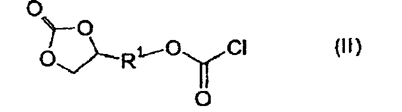 dans laquelle R¹ a la signification mentionnée pour la formule générale (I)
avec un composé de formule générale (III) 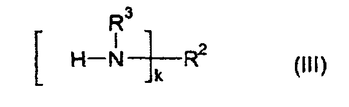 dans laquelle k ainsi que R² et R³, en fonction de k, présentent les significations décrites pour la formule générale (I) ou un sel de ceux-ci.

2. Procédé selon la revendication 1, où des composés de formule générale (II) sont utilisés, dans laquelle R¹ représente un radical C₁-C₆-alkylène, de préférence un radical méthylène, éthylène, n-propylène, n-butylène ou n-hexaméthylène.

3. Procédé selon la revendication 1 ou 2, où les composés de formule générale (III) sont utilisés sous forme des sels d'ammonium de formule générale (IIIA), 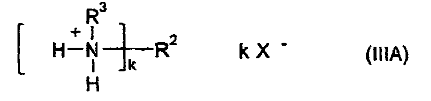 où R² et R³ présentent à chaque fois les significations mentionnées dans le contexte de la formule (III) et X représente un halogénure, de préférence le chlore, le brome ou l'iode ou méthylsulfate.

4. Procédé selon l'une ou plusieurs des revendications 1 à 3, où des composés de formule générale (III) sont utilisés, dans laquelle
k vaut 1 et
R² et R³ sont identiques ou différents et représentent C₁-C₃₀-alkyle, C₁-C₆-alkyloxy-C₁-C₃₀-alkyle, C₁-C₆- alkylcarbonyloxy-C₁-C₃₀-alkyle, amino-C₁-C₃₀-alkyle, mono(C₁-C₆-alkyl)amino-C₁-C₃₀-alkyle ou di(C₁-C₆- alkyl)amino-C₁-C₃₀-alkyle, mono(C₇-C₁₁- aralkyl)amino-C₁-C₃₀-alkyle ou di(C₇-C₁₁- aralkyl)amino-C₁-C₃₀-alkyle, mono(allyl)amino-C₁- C₃₀-alkyle ou di(allyl)amino-C₁-C₃₀-alkyle, ammonio- C₁-C₃₀-alkyle, polyoxyalkylène-C₁-C₃₀-alkyle, polysiloxanyl-C₁-C₃₀-alkyle (méth)acryloyloxy-C₁- C₃₀-alkyle, 3-imidazolio-C₁-C₃₀-alkyle ou [C₂-C₆- alkylène-O]ₓ-[C₂-C₆-alkylène-O]_{y}-C₁-C₆-alkyle, où x = 0-50 et y = 0-40, soit x soit y vaut au moins 1 et les deux radicaux alkylène sont différents lorsque x et y sont tous les deux différents de zéro, ou
R³ peut également représenter hydrogène.

5. Procédé selon l'une ou plusieurs des revendications 1 à 4, où des composés de formule générale (III) sont utilisés, dans laquelle
k vaut 1 et
R² représente C₁-C₁₈-alkyle, C₁-C₆-alkyloxy-C₁-C₁₈- alkyle, C₁-C₆-alkylcarbonyloxy-C₁-C₁₈-alkyle, amino- C₁-C₁₈-alkyle, mono(C₁-C₆-alkyl)amino-C₁-C₁₈-alkyle ou di(C₁-C₆-alkyl)amino-C₁-C₁₈-alkyle, mono(C₇-C₁₁- aralkyl)amino-C₁-C₁₈-alkyle ou di(C₇-C₁₁- aralkyl)amino-C₁-C₁₈-alkyle, mono(allyl)amino-C₁- C₁₈-alkyle ou di(allyl)amino-C₁-C₁₈-alkyle, ammonio- C₁-C₁₈-alkyle, polyoxyalkylène-C₁-C₁₈-alkyle, polysiloxanyl-C₁-C₁₈-alkyle, (méth)acryloyloxy-C₁- C₁₈-alkyle, 3-imidazolio-C₁-C₃₀-alkyle ou [C₂-C₆- alkylène-O]ₓ- [C₂-C₆-alkylène-O]_{y}-C₁-C₆-alkyle, où les deux radicaux alkylène sont différents et x = 1-50 et y = 1-40, et
R³ signifie hydrogène.

6. Procédé selon l'une ou plusieurs des revendications 1 à 5, où des composés de formule générale (III) sont utilisés, dans laquelle
k vaut 1 et
R² et R³ sont identiques ou différents et représentent C₁-C₁₈-alkyle, amino-C₁-C₁₈-alkyle, mono(C₁-C₆- alkyl)amino-C₁-C₁₈-alkyle ou di(C₁-C₆-alkyl)amino- C₁-C₁₈-alkyle, mono(C₇-C₁₁-aralkyl)amino-C₁-C₁₈- alkyle ou di(C₇-C₁₁-aralkyl)amino-C₁-C₁₈-alkyle, mono(allyl)amino-C₁-C₁₈-alkyle ou di(allyl)amino-C₁- C₁₈-alkyle, ammonio-C₁-C₁₈-alkyle, polyoxyalkylène- C₁-C₁₈-alkyle, polysiloxanyl-C₁-C₁₈-alkyle, 3- imidazolio-C₁-C₃₀-alkyle ou [C₂-C₆-alkylène-O]ₓ-[C₂- C₆-alkylène-O]_{y}-C₁-C₆-alkyle, où les deux radicaux alkylène sont différents et x = 2-40 et y = 2-20.

7. Procédé selon l'une ou plusieurs des revendications 1 à 3, où des composés de formule générale (III) sont utilisés, dans laquelle
k vaut 1,
R³ signifie hydrogène et
R² représente -(CH₂)ₙ-CH₃,
-(CH₂)ₙ-CH((CH₂)ₘ-CH₃)-(CH₂)ₒ-CH₃, où n + m + o vaut
au maximum 27,
- (CH₂)ₙ-(CF₂)ₚ-CF₃,
- (CH₂)ₙ-[O-Si(-O-Si(CH₃)₃)₃],
- (CH₂)ₙ-(O-CH₂-CH₂)_{q}-O-R⁴-H,
-R⁴-OH,
-R⁴-NR⁵₂,
-R⁴-NR⁵₃⁺ Y⁻,
-R₄-OC(=O)-CR⁶=CH₂,
-R⁴-SO₃H,
-R⁴-PO₃H₂,
-R⁴-OPO₃H₂
ou un radical saccharide,
où
R⁴ représente C₁-C₆-alkylène,
R⁵ représente hydrogène, C₁-C₁₈-alkyle, C₂-C₁₈- alcényle, de préférence allyle, ou benzyle,
R⁶ représente hydrogène ou méthyle et
Y représente un équivalent d'un anion, de préférence chlorure, bromure, sulfate, hydrogénosulfate, méthosulfate ou nitrate,
n, m, o valent, indépendamment l'un de l'autre, un nombre entier de 0 à 17,
p vaut un nombre entier de 1 à 12 et
q représente un nombre entier de 1 à 100, de préférence de 2 à 50.

8. Procédé selon l'une ou plusieurs des revendications 1 à 3, où des composés de formule générale (III) sont utilisés, dans laquelle
k vaut un nombre entier supérieur à 1, en particulier 2 ou 3,
R² représente un radical hydrocarboné de valence k, en particulier divalent ou trivalent, aliphatique, cycloaliphatique, aromatique ou araliphatique, qui contient le cas échéant un ou plusieurs hétéroatomes, et
R³ représente hydrogène, C₁-C₃₀-alkyle, C₁-C₆-alkyloxy- C₁-C₃₀-alkyle, C₁-C₆-alkylcarbonyloxy-C₁-C₃₀-alkyle, amino-C₁-C₃₀-alkyle, mono(C₁-C₆-alkyl)-amino-C₁-C₃₀- alkyle ou di(C₁-C₆-alkyl)-amino-C₁-C₃₀-alkyle, mono(C₇-C₁₁-aralkyl)amino-C₁-C₃₀-alkyle ou di(C₇-C₁₁- aralkyl)amino-C₁-C₃₀-alkyle, mono(allyl)amino-C₁- C₃₀-alkyle ou di(allyl)amino-C₁-C₃₀-alkyle, ammonio- C₁-C₃₀-alkyle, polyoxyalkylène-C₁-C₃₀-alkyle, polysiloxanyl-C₁-C₃₀-alkyle, (méth)acryloyloxy-C₁- C₃₀-alkyle, 3-imidazolio-C₁-C₃₀-alkyle ou [C₂-C₆- alkylène-O]ₓ-[C₂-C₆-alkylène-O]_{y}-C₂-C₆-alkyle, où x = 0-50 et y = 0-50, mais ou soit x soit y vaut au moins 1 et les deux radicaux alkylène sont différents lorsque x et y sont différents de 0.

9. Procédé selon l'une ou plusieurs des revendications 1 à 3 ou 8, où des composés de formule générale (III) sont utilisés, dans laquelle
k vaut 2 ou 3,
R² représente un radical hydrocarboné divalent ou trivalent, aliphatique, cycloaliphatique, aromatique ou araliphatique, qui contient le cas échéant exclusivement un ou plusieurs atomes d'oxygène comme hétéroatomes, et
R³ représente hydrogène, C₁-C₁₈-alkyle, C₁-C₆-alkyloxy- C₁-C₁₈-alkyle, C₁-C₆-alkylcarbonyloxy-C₁-C₁₈-alkyle, amino-C₁-C₁₈-alkyle, mono(C₁-C₆-alkyl)-amino-C₁-C₁₈- alkyle ou di(C₁-C₆-alkyl)-amino-C₁-C₁₈-alkyle, ammonio-C₁-C₁₈-alkyle, polyoxyalkylène-C₁-C₁₈- alkyle, polysiloxanyl-C₁-C₁₈-alkyle, (méth)acryloyloxy-C₁-C₁₈-alkyle, 3-imidazolio-C₁- C₃₀-alkyle ou [C₂-C₆-alkylène-O]ₓ-[C₂-C₆-alkylène- O]_{y}-C₂-C₆-alkyle, où x = 1-50 et y = 1-50 et les deux radicaux alkylène sont différents.

10. Procédé selon l'une ou plusieurs des revendications 1 à 3 et 8 à 9, où des composés de formule générale (III) sont utilisés, dans laquelle
k vaut 2,
R² représente un groupe -[(C₂-C₆-alkylène-O-)ₓ-(C₂-C₆- alkylène)]-, où les deux groupes alkylène sont identiques ou différents et x = 1-50, de préférence x = 1-40, ou un groupe -[(C₂-C₆- alkylène-O-)ₓ-(C₂-C₆-alkylène-O-)_{y}-(C₂-C₆-alkylène- O-)_{z}-C₂-C₆-alkylène]-, où les trois unités alkylène sont différentes ou la première peut être identique à la troisième, et où x = 0-50, en particulier x = 1-40, y = 1-50, en particulier y = 1-40, et z = 1-50, en particulier z = 1-40 et
R³ signifie hydrogène ou C₁-C₃₀-alkyle.

11. Procédé selon l'une ou plusieurs des revendications 1 à 3, où, pour le cas où k = 1, on utilise comme composés de formule générale (III) des polyétheramines, la n-octylamine, la tert-octylamine, la n-hexylamine, la 2-éthylhexylamine, la taurine, la dihexylamine, la dodécylamine, l'octadécylamine, la méthyloctadécylamine, la dioctylamine, la N,N-diméthylpropylènediamine, la N,N-diméthyléthylènediamine, l'iodure de 3-aminopropyltriméthylammonium, le bromure de 3-aminopropyltriméthylammonium, le chlorure de 3-aminopropyltriméthylammonium, l'iodure de 3-aminoéthyltriméthylammonium, le bromure de 3-aminoéthyltriméthylammonium, le chlorure de 3-aminoéthyltriméthylammonium, le chlorure de 3-aminopropyldiméthyloctylammonium, le bromure de 3-aminopropyldiméthyloctylammonium, la 3-(triméthylsilyl)-propylamine, la 3-(tris(triméthylsilyloxy)silyl)-propylamine, la 3-(triméthylsilyl)-éthylamine, la 3-(tris(triméthylsilyloxy)silyl)-éthylamine, le 3-aminopropylimidazole, le 2-aminoéthylimidazole, le 4-aminobutylimidazole, le méthylsulfate de 3-aminopropyl-1-méthylimidiazolium, le méthylsulfate de 3-aminopropyl-1-butylimidiazolium, le méthylsulfate de 3-aminoéthyl-1-méthylimidiazolium, le méthylsulfate de 3-aminoéthyl-1-butylimidiazolium, le chlorure de 3-aminopropyl-1-méthylimidiazolium, le chlorure de 3-aminopropyl-1-butylimidiazolium, le bromure de 3-aminopropyl-1-méthylimidiazolium, le bromure de 3-aminopropyl-1-butylimidiazolium, le chlorure de 3-aminoéthyl-1-méthylimidiazolium, le chlorure de 3-aminoéthyl-1-butylimidiazolium, le bromure de 3-aminoéthyl-1-méthylimidiazolium ou le bromure de 3-aminoéthyl-1-butylimidiazolium.

12. Procédé selon l'une ou plusieurs des revendications 1 à 3, où on utilise comme composés de formule générale (III), pour le cas où k est un nombre entier supérieur à 1, des polyétherdiamines, l'éthylènediamine, la propylènediamine, la butylènediamine, l'hexaméthylènediamine, la MDA, la TDA, le diaminobenzène, le diaminocyclohexane, le diaminométhylcyclohexane, la TCD-diamine, le 1,4-bis-(3-aminopropyloxy)butane, la tris-(2-aminoéthyl)amine, la 1,4-bis-(3-aminopropyl)pipérazine, la bis-(3-aminopropyl)amine ou la triéthylènetétraamine.

13. Procédé selon l'une ou plusieurs des revendications 1 à 12, où on utilise en outre une base auxiliaire, de préférence la triméthylamine, la triéthylamine, la tripropylamine, la triisopropylamine, la tributylamine, la pyridine, la 1,2-diazine, la 1,3-diazine, la 1,4-diazine, la 1,2,3-triazine, la 1,2,4-triazine, la 1,3,5-triazine, le C₁-C₃₀-alkylpyrrole, l'isoazole, l'oxazole, l'isothiazole, le thiazole, le C₁-C₃₀-alkylpyrazole, le C₁-C₃₀-alkylimidazole, le 1,2,4-thiadiazole, le C₁-C₃₀-alkyl-1,2,3-triazole, le C₁-C₃₀-alkyl-1,2,4-triazole, la diméthylaniline, la diméthylaminopyridine, le carbonate de sodium, l'hydrogénocarbonate de sodium, le carbonate de potassium, l'hydrogénocarbonate de potassium, l'hydroxyde de sodium ou l'hydroxyde de potassium.

14. Procédé selon l'une ou plusieurs des revendications 1 à 13, où on utilise, par rapport au composé utilisé de formule générale (III) 0,75 à 4 équivalents du chloroformiate de formule générale (II), de préférence 0,9 à 3 équivalents, de manière particulièrement préférée 1,0 à 2,0 équivalents.

15. Procédé selon l'une ou plusieurs des revendications 1 à 14, où le procédé est réalisé à une température dans la plage de -10°C à 100°C, de préférence à une température dans la plage de -5°C à 80°C, de manière particulièrement préférée dans la plage de 0°C à 60°C et en particulier dans la plage de 0°C à 40°C.
